# EUROPÄISCHE PATENTSCHRIFT

(11) **EP 2 456 415 B1**
(45) Veröffentlichungstag und Bekanntmachung des Hinweises auf die Patenterteilung: **05.07.2017**
(21) Anmeldenummer: 10731759.6
(22) Anmeldetag: 16.07.2010
(51) Int. Cl.: A61K 8/37, A61K 8/81, A61Q 5/06

(54) **MITTEL FÜR KERATINHALTIGE FASERN, ENTHALTEND MINDESTENS EIN ACRYLAT/GLYCERYLACRYLAT-COPOLYMER, MINDESTENS EIN FILMBILDENDES UND/ODER FESTIGENDES POLYMER UND MINDESTENS EIN ESTERÖL**
AGENT FOR KERATINOUS FIBERS, COMPRISING AT LEAST ONE ACRYLATE/GLYCERYL ACRYLATE COPOLYMER, AT LEAST ONE FILM-FORMING AND/OR SOLIDIFYING POLYMER AND AT LEAST ONE ESTER OIL
AGENTS POUR FIBRES KÉRATINIQUES, CONTENANT AU MOINS UN COPOLYMÈRE ACRYLATE/GLYCÉRYLACRYLATE, AU MOINS UN POLYMÈRE FILMOGÈNE ET/OU RENFORÇATEUR ET AU MOINS UNE HUILE-ESTER

(30) Priorität: 22.07.2009 DE 102009027925
(43) Veröffentlichungstag der Anmeldung: 30.05.2012
(73) Patentinhaber: Henkel AG & Co. KGaA, 40589 Düsseldorf (DE)
(72) Erfinder: KNAPPE, Thorsten, 22869 Schenefeld (DE); FLODROP VAN, Helga, 21075 Hamburg (DE)
(86) Internationale Anmeldenummer: PCT/EP2010/060297
(87) Internationale Veröffentlichungsnummer: WO 2011/009815

(56) Entgegenhaltungen:
- EP-B1- 1 028 698
- WO-A1-94/08555
- WO-A1-2005/016291
- WO-A2-2010/059458
- "A new high-spreading ester emollient for the hair care market", RESEARCH DISCLOSURE, MASON PUBLICATIONS, HAMPSHIRE, GB, Bd. 510, Nr. 31, 1. Oktober 2006 (2006-10-01), Seite 1302, XP007136710, ISSN: 0374-4353
- "A new high-spreading ester emollient for hair care applications", RESEARCH DISCLOSURE, MASON PUBLICATIONS, HAMPSHIRE, GB, Bd. 524, Nr. 4, 1. Dezember 2007 (2007-12-01), Seite 1182, XP007137786, ISSN: 0374-4353

## Beschreibung

Die vorliegende Erfindung betrifft die Verwendung eines Mittels zur temporären Verformung von Haaren enthaltend ein Copolymerisat aus Glycerylacrylat und Acrylsäure, mindestens eines filmbildenden und/oder festigenden Polymers und mindestens eines Esteröls. Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere, sogenannte festigende Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Eine wichtige Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt.

Stylingmittel zu entwickeln, die alle gewünschten Eigenschaften in Kombination aufweisen, bereitet nach wie vor Schwierigkeiten. Insbesondere gilt dies für die Kombination von ästhetischen Gesichtpunkten einerseits und starkem und flexiblem Halt andererseits. Um einen starken Halt zu vermitteln muss das fixierend wirkende Polymer gut auf der keratinhaltigen Faser haften und einen hinreichend harten Film bilden. Dennoch der resultierende Polymerfilm dem Faserkollektiv keine Taktilität eines Bretts verleihen, sondern den Fasern einen Grad an Flexibilität ermöglichen, ohne dass die aufgeprägte Formgebung des Faserkollektivs, d.h. z.B. eine Frisur, verloren geht.

Kosmetische Mittel, die neben weiteren Bestandteilen ein Copolymer mit der INCI-Bezeichnung Glycerylpolymethacrylate, ein filmbildendes und/oder festigendes Polymer sowie ein Esteröl enthalten, werden in der internationalen Patentanmeldung WO 2005/016291 A1 beschrieben.

Die Veröffentlichungen "A new high-spreading ester emollient for the hair care market" (Research Disclosure, Mason Publications, Hampshire, GB, Bd. 510, Nr. 31, 1, Seite 1302) und "A new high-spreading ester emollient for hair care applications" (Research Disclosure, Mason Publications, Hampshire, GB, Bd. 524, Nr. 4, 1, Seite 1182) beschreiben Stylingmittel zur temporären Haarverformung. Diese Stylingmitteln enthalten neben anderen Inhaltsstoffen Copolymer mit der INCI-Bezeichnung Glycerylpolyacrylate, filmbildendes und/oder festigendes Polymer sowie Esteröl.

Aufgabe der vorliegenden Erfindung war es daher, ein Mittel zur temporären Verformung keratinischer Fasern zur Verfügung zu stellen, das sich durch einen hohen Haltegrad auszeichnet und hervorragende Pflegeeigenschaften der keratinhaltigen Faser ermöglicht.

Es wurde nunmehr überraschenderweise gefunden, dass dies durch die Kombination von Inhaltsstoffen (*vide infra*) erreicht werden kann. Die Mittel lassen sich hervorragend auf dem Haar verteilen, verleihen einen perfekten Frisurenhalt und gleichmäßig gepflegte Keratinfasern.

Ein erster Gegenstand der vorliegenden Erfindung ist daher die Verwendung eines Mittels, enthaltend in einem kosmetisch akzeptablen Träger
(a) mindestens ein Copolymerisat aus Glycerylacrylat und Acrylsäure
   und
(b) mindestens ein filmbildendes und/oder festigendes Polymer
   und
(c) mindestens ein Esteröl.
zur temporären Verformung von Haaren.

Die Copolymere der Komponente (a) sind in dem erfindungsgemäßen Mittel bevorzugt in einer Menge von 0,001 Gew.-% bis 1,0 Gew.-%, besonders bevorzugt von 0,005 Gew.-% bis 0,5 Gew.-%, ganz besonders bevorzugt von 0,005 bis 0,1 Gew.%, jeweils bezogen auf das Gewicht des Mittels, enthalten.

Das Mittel enthält zwingend zusätzlich zum Copolymer (a) und dem Esteröl (c) mindestens ein filmbildendes und/oder festigendes Polymer. Dieses ist von den Copolymeren (a) verschieden.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Als eine Testmethode für die festigende Wirkung eines Polymers wird häufig der so genannte curl-retention - Test angewendet.

Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch.

Die filmbildenden und/oder festigenden Polymere sind in dem erfindungsgemäßen Mittel bevorzugt in einer Menge von 0,01 Gew.-% bis 20 Gew.-%, insbesondere von 0,5 Gew.-% bis 15 Gew.-%, ganz besonders bevorzugt von 2,0 Gew.-% bis 10,0 Gew.-%, jeweils bezogen auf das Gewicht des Mittels, enthalten. Diese Mengenangaben gelten auch für alle in den erfindungsgemäßen Mitteln einsetzbaren nachfolgenden bevorzugten Typen der filmbildenden und/oder festigenden Polymere. Falls nachfolgend abweichende bevorzugte Mengen spezifiziert wurden, gelten letztere als wiederum noch bevorzugter Mengen.

Die pulverförmige Zusammensetzung enthält bevorzugt als filmbildendes und/oder festigendes Polymer
- mindestens ein kationisches filmbildendes und/oder kationisches festigendes Polymer und/oder
- mindestens ein nichtionisches filmbildendes und/oder nichtionisches festigendes Polymer und/oder
- mindestens ein anionisches filmbildendes und/oder anionisches festigendes Polymer und/oder
- mindestens ein amphoteres filmbildendes und/oder amphoteres festigendes Polymer.

Die pulverförmige Zusammensetzung enthält besonders bevorzugt als filmbildendes und/oder festigendes Polymer
- mindestens ein kationisches filmbildendes und/oder kationisches festigendes Polymer und/oder
- mindestens ein nichtionisches filmbildendes und/oder nichtionisches festigendes Polymer.

Die pulverförmigen Zusammensetzungen enthalten als filmbildendes und/oder festigendes Polymer mindestens ein kationisches filmbildendes und/oder kationisches festigendes Polymer.

Die kationischen filmbildenden und/oder kationischen festigenden Polymere weisen mindestens eine Struktureinheit auf, die mindestens ein permanent kationisiertes Stichstoffatom enthält. Unter permanent kationisierten Stickstoffatomen sind solche Stickstoffatome zu verstehen, die eine positive Ladung tragen und dadurch eine quartäre Ammoniumverbindung bilden. Quartäre Ammonium-Verbindungen werden meist durch Umsetzung tertiärer Amine mit Alkylierungsmitteln, wie z.B. Methylchlorid, Benzylchlorid, Dimethylsulfat, Dodecylbromid, aber auch Ethylenoxid hergestellt. In Abhängigkeit von dem eingesetzten tertiären Amin sind insbesondere folgende Gruppen bekannt: Alkylammonium-Verbindungen, Alkenylammonium-Verbindungen, Imidazolinium-Verbindungen und Pyridinium-Verbindungen. Bevorzugte pulverförmige Zusammensetzungen enthalten die filmbildenden kationischen und/oder festigenden kationischen Polymere in einer Menge von 0,1 Gew.-% bis 8,0 Gew.-%, besonders bevorzugt von 0,2 Gew.-% bis 7,0 Gew.-%, ganz besonders bevorzugt von 0,5 Gew.-% bis 5,0 Gew.-%, jeweils bezogen auf das Gewicht der Zusammensetzung.

Die kationischen filmbildenden und/oder kationischen festigenden Polymere können aus kationischen, quaternisierten Cellulose-Derivaten ausgewählt werden. Es erweisen sich generell solche kationischen, quaternisierten Cellulosen als im Sinne der Erfindung vorteilhaft, die in einer Seitenkette mehr als eine permanente kationische Ladung tragen.

Darunter sind unter den kationischen Cellulosederivaten solche hervorzuheben, die aus Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat^{®} H 100, Celquat^{®} L 200 von der Firma National Starch vertrieben werden.

Weiterhin eignen sich solche kationischen filmbildenden und/oder kationischen festigenden Polymere, die mindestens eine Struktureinheit der Formel (M-I) und mindestens eine Struktureinheit der Formel (M-VI) und gegebenenfalls mindestens eine Struktureinheit der Formel (M-V) umfassen 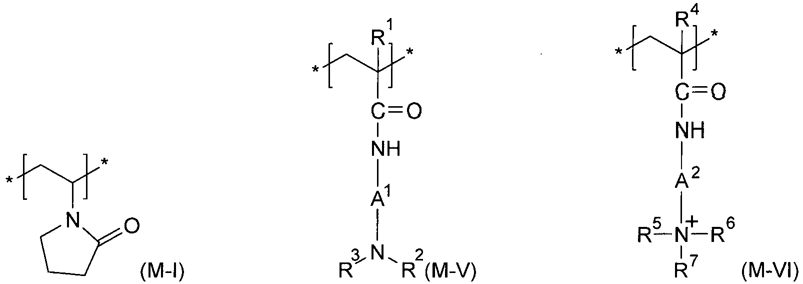 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

Zur Kompensation der positiven Ladung des Monomers (M-VI) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Geeignete Verbindungen sind beispielsweise als
- Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat^{®} 440, Gafquat^{®}734, Gafquat^{®}755 (jeweils Firma ISP) sowie Luviquat PQ 11 PN (Firma BASF SE),
- Copolymere aus Methacryloylaminopropyllauryldimethylammonium chlorid mit Vinylpyrrolidon und Dimethylaminopropylmethacrylamid mit der INCI-Bezeichnung Polyquaternium-55 unter den Handelsnamen, Styleze^{®} W-10, Styleze^{®} W-20 (Firma ISP),
- Copolymere aus N-Vinylpyrrolidon, N-Vinylcaprolactam, N-(3-Dimethylaminopropyl)-methacrylamid und 3-(Methacryloylamino)propyl-lauryl-dimethylammoniumchlorid (INCI-Bezeichnung: Polyquaternium-69) unter dem Handelsnamen AquaStyle^{®} 300 (28-32 Gew.-% Aktivsubstanz in Ethanol-Wasser-Gemisch) (Firma ISP)
im Handel erhältlich.

Als besonders bevorzugt verwendbare filmbildende und/oder festigende Polymere ausgewählt aus kationischen Polymeren die mindestens eine Struktureinheit enthalten, die ein permanent kationisiertes Stickstoffatom aufweisen, dienen weiterhin solche kationischen filmbildenden und/oder kationischen festigenden Polymere, die mindestens ein Strukturelement der Formel (M1) enthalten 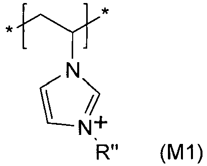 worin
R" für eine (C₁ bis C₄)-Alkylgruppe, insbesondere eine Methylgruppe, steht,
und zusätzlich mindestens ein weiteres kationisches und/oder nichtionisches Strukturelement aufweisen.

Zur Kompensation der positiven Polymerladung der Komponente dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist wiederum bevorzugt, wenn in der pulverförmigen Zusammensetzung als kationisches filmbildendes und/oder kationisches festigendes Polymer dieser Ausführungsform, mindestens ein Copolymer (b1) enthalten ist, das neben mindestens einem Strukturelement der Formel (M1) zusätzlich ein Strukturelement der Formel (M-I) umfasst 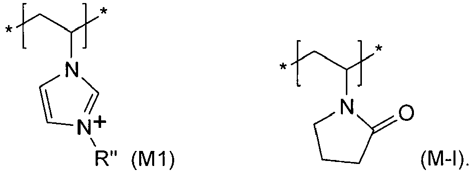 worin
R" für eine (C₁ bis C₄)-Alkylgruppe, insbesondere eine Methylgruppe, steht.

Zur Kompensation der positiven Polymerladung der Copolymere (b1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische filmbildende und/oder kationische festigende Polymere als Copolymere (b1) enthalten 10 bis 30 Mol-%, vorzugsweise 15 bis 25 Mol.-% und insbesondere 20 Mol.-% Struktureinheiten gemäß Formel (M1) und 70 bis 90 Mol.-%, vorzugsweise 75 bis 85 Mol.-% und insbesondere 80 Mol.-% Struktureinheiten gemäß Formel (M-I).

Hierbei ist besonders bevorzugt, wenn die Copolymere (b1) neben Polymereinheiten, die aus dem Einbau der genannten Struktureinheiten gemäß Formel (M1) und (M-I) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b1) ausschließlich aus Struktureinheiten der Formel (M1) mit R" = Methyl und (M-I) aufgebaut und lassen sich durch die allgemeine Formel (Poly1) 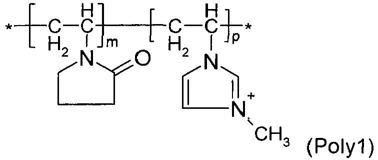 beschreiben, wobei die Indices m und p je nach Molmasse des Polymers variieren und nicht bedeuten sollen, daß es sich um Blockcopolymere handelt. Vielmehr können Struktureinheiten der Formel (M1) und der Formel (M-I) im Molekül statistisch verteilt vorliegen.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat^{®} UltraCare erhältlich.

Besonders bevorzugte pulverförmige Zusammensetzungen enthalten ein Copolymer (b1), insbesondere der Formel (Poly1), das Molmassen innerhalb eines bestimmten Bereiches aufweist. Hier sind erfindungsgemäße Mittel bevorzugt, bei denen das Copolymer (b1) eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) (b1) oder an dessen bzw. deren Stelle können erfindungsgemäße pulverförmige Zusammensetzungen auch Copolymere (b2) enthalten, die ausgehend vom Copolymer (b1) als zusätzliche Struktureinheiten Struktureinheiten der Formel (M-II) aufweisen

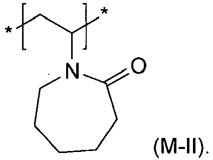

Weitere besonders bevorzugte pulverförmige Zusammensetzungen sind somit dadurch gekennzeichnet, daß sie als kationisches filmbildendes und/oder kationisches festigendes Polymer mindestens ein Copolymer (b2) enthalten, das mindestens eine Struktureinheit gemäß Formel (M1-a) und mindestens eine Struktureinheit gemäß Formel (M-I) und mindestens eine Struktureinheit gemäß Formel (M-II) enthält

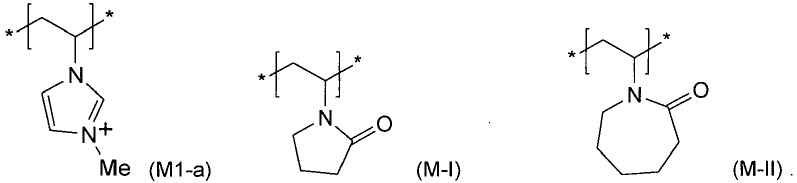

Auch hierbei ist besonders bevorzugt, wenn die Copolymere (b2) neben Polymereinheiten, die aus dem Einbau der genannten Struktureinheiten gemäß Formel (M1-a), (M-I) und (M-II) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b2) ausschließlich aus Struktureinheiten der Formeln (M1-a), (M-I) und (M-II) aufgebaut und lassen sich durch die allgemeine Formel (Poly2) 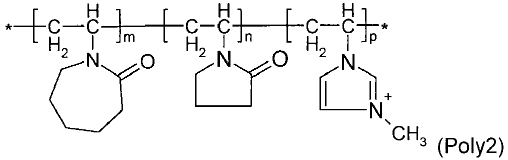 beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen.

Zur Kompensation der positiven Polymerladung der Komponente (b2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (b2) enthalten 1 bis 20 Mol-%, vorzugsweise 5 bis 15 Mol.-% und insbesondere 10 Mol.-% Struktureinheiten gemäß Formel (M1-a) und 30 bis 50 Mol.-%, vorzugsweise 35 bis 45 Mol.-% und insbesondere 40 Mol.-% Struktureinheiten gemäß Formel (I) und 40 bis 60 Mol.-%, vorzugsweise 45 bis 55 Mol.-% und insbesondere 60 Mol.-% Struktureinheiten gemäß Formel (M-II).

Besonders bevorzugte pulverförmige Zusammensetzungen enthalten ein Copolymer (b2), das Molmassen innerhalb eines bestimmten Bereiches aufweist. Hier sind erfindungsgemäße Mittel bevorzugt, bei denen das Copolymer (b2) eine Molmasse von 100 bis 1000 kDa, vorzugsweise von 250 bis 900 kDa, weiter bevorzugt von 500 bis 850 kDa und insbesondere von 650 bis 710 kDa aufweist.

Zusätzlich zu dem bzw. den Copolymer(en) (b1) und/oder (b2) oder an dessen bzw. deren Stelle können die pulverförmigen Zusammensetzungen als filmbildendes kationische und/oder festigendes kationisches Polymer auch Copolymere (b3) enthalten, die als Struktureinheiten Struktureinheiten der Formeln (M1-a) und (I) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte pulverförmige Zusammensetzungen sind dadurch gekennzeichnet, daß sie als kationisches filmbildendes und/oder kationisches festigendes Polymer mindestens ein Copolymer (b3) enthalten, das mindestens eine Struktureinheit gemäß Formel (M1-a) und mindestens eine Struktureinheit gemäß Formel (M-I) und mindestens eine Struktureinheit gemäß Formel (M-VII) und mindestens eine Struktureinheit gemäß Formel (M-VIII) enthält

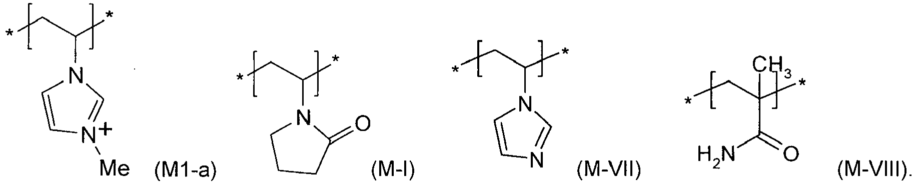

Auch hierbei ist besonders bevorzugt, wenn die Copolymere (b3) neben Polymereinheiten, die aus dem Einbau der genannten Struktureinheiten gemäß Formel (M1-a), (M-I), (M-VII) und (M-VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b3) ausschließlich aus Struktureinheiten der Formel (M1-a), (M-I), (M-VII) und (M-VIII) aufgebaut und lassen sich durch die allgemeine Formel (Poly3) 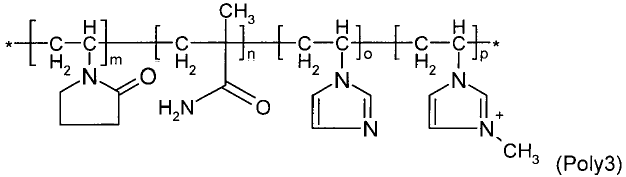 beschreiben, wobei die Indices m, n, o und p je nach Molmasse des Polymers variieren und nicht bedeuten sollen, daß es sich um Blockcopolymere handelt. Vielmehr können Struktureinheiten der Formeln (M1-a), (M-I), (M-VII) und (M-VIII) im Molekül statistisch verteilt vorliegen.

Zur Kompensation der positiven Polymerladung der Komponente (b2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (b3) enthalten 1 bis 12 Mol-%, vorzugsweise 3 bis 9 Mol.-% und insbesondere 6 Mol.-% Struktureinheiten gemäß Formel (M1-a) und 45 bis 65 Mol.-%, vorzugsweise 50 bis 60 Mol.-% und insbesondere 55 Mol.-% Struktureinheiten gemäß Formel (M-I) und 1 bis 20 Mol.-%, vorzugsweise 5 bis 15 Mol.-% und insbesondere 10 Mol.-% Struktureinheiten gemäß Formel (M-VII) und 20 bis 40 Mol.-%, vorzugsweise 25 bis 35 Mol.-% und insbesondere 29 Mol.-% Struktureinheiten gemäß Formel (M-VIII).

Besonders bevorzugte Mittel enthalten ein Copolymer (b3), das Molmassen innerhalb eines bestimmten Bereiches aufweist. Hier sind erfindungsgemäße Mittel bevorzugt, bei denen das Copolymer (b3) eine Molmasse von 100 bis 500 kDa, vorzugsweise von 150 bis 400 kDa, weiter bevorzugt von 250 bis 350 kDa und insbesondere von 290 bis 310 kDa aufweist.

Unter den zusätzlichen filmbildenden kationischen und/oder festigenden Polymer ausgewählt aus den kationischen Polymeren mit mindestens einem Strukturelement der obigen Formel (M1), gelten als bevorzugt:
- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat^{®} Style, Luviquat^{®} FC 370, Luviquat^{®} FC 550, Luviquat^{®} FC 905 und Luviquat^{®} HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat^{®} Care (BASF SE)),
- Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat^{®} Care oder Luviquat^{®} Hold (BASF SE)),
- Vinylpyrrolidon/Methacrylamid/Vinylimidazol/1-Vinyl-3-methyl-1 H-imidazoliummethylsulfat-Copolymer (wie beispielsweise das mit der INCI-Bezeichnung

Polyquaternium-68 unter der Handelsbezeichnung Luviquat^{®} Supreme (BASF SE)), sowie Gemische aus diesen Polymeren.

Im Rahmen einer bevorzugten Ausführungsform enthalten die pulverförmigen Zusammensetzungen als filmbildendes und/oder festigendes Polymer mindestens ein filmbildendes nichtionisches und/oder festigendes nichtionisches Polymer.

Unter einem nichtionischen Polymer wird ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Die filmbildenden nichtionischen und/oder festigenden nichtionischen Polymere sind in dem erfindungsgemäßen Mittel bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 5,0 Gew.-%, jeweils bezogen auf das Gewicht der erfindungsgemäßen pulverförmigen Zusammensetzung, enthalten.

Die filmbildenden nichtionischen und/oder festigenden nichtionischen Polymere werden wiederum bevorzugt ausgewählt aus mindestens einem Polymer der Gruppe, die gebildet wird, aus
- Homopolymeren und nichtionischen Copolymeren des N-Vinylpyrrolidons,
- nichtionischen Copolymeren des Isobutens,
- nichtionischen Copolymeren des Maleinsäureanhydrids.

Dabei ist eine Kombination von filmbildenden nichtionischen und/oder festigenden nichtionischen Polymeren bevorzugt, umfassend mindestens ein nichtionisches Copolymer des Maleinsäureanhydrids und mindestens ein Polymer aus der Gruppe, die gebildet wird aus Homopolymeren und nichtionischen Copolymeren des N-Vinylpyrrolidons.

Geeignete Polyvinylpyrrolidone sind beispielsweise Handelsprodukte wie Luviskol^{®} K 90 oder Luviskol^{®} K 85 der Firma BASF SE.

Geeignete Polyvinylalkohole werden beispielsweise unter den Handelsbezeichnungen Elvanol^{®} von Du Pont oder Vinol^{®} 523/540 von der Firma Air Products vertrieben.

Geeignetes Polyvinylacetat wird beispielsweise unter dem Handelsnamen Vinac^{®} als Emulsion von der Firma Air Products vertrieben.

Mittel, die als filmbildendes nichtionisches und/oder festigendes nichtionisches Polymer mindestens ein Polymer ausgewählt aus der Gruppe, die gebildet wird aus
- Copolymer aus Maleinsäureanhydrid und Methylvinylether,
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
- Copolymere aus N-Vinylpyrrolidon mit N,N-Di(C₁ bis C₄)-Alkylamino-(C₂ bis C₄)-alkylacrylamid,
oder Gemische aus diesen Polymeren enthalten, sind erfindungsgemäß ganz besonders bevorzugt.

Dabei ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt.

Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Weitere bevorzugte pulverförmige Zusammensetzungen sind dadurch gekennzeichnet, daß sie als nichtionisches filmbildendes und/oder nichtionisches festigendes Polymer mindestens ein Copolymer (n1) enthalten, das mindestens eine Struktureinheit gemäß Formel (M-I) und mindestens eine Struktureinheit gemäß Formel (M-VII) enthält und mindestens eine Struktureinheit gemäß Formel (M-VIII) enthält

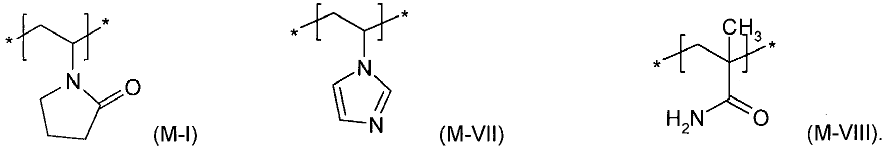

Auch hierbei ist besonders bevorzugt, wenn diese Copolymere neben Polymereinheiten, die aus dem Einbau der genannten Struktureinheiten gemäß Formel (M1-a), (I), (VII) und (VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (n1) ausschließlich aus Struktureinheiten der Formel (M1-a), (I), (VII) und (VIII) aufgebaut und lassen sich durch die allgemeine Formel (Poly4) 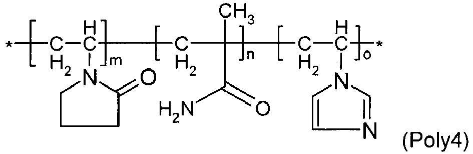 beschreiben, wobei die Indices m, n, o und p je nach Molmasse des Polymers variieren und nicht bedeuten sollen, daß es sich um Blockcopolymere handelt. Vielmehr können Struktureinheiten der Formeln (I), (VII) und (VIII) im Molekül statistisch verteilt vorliegen.

Ein besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Weiterhin eignen sich solche pulverförmigen Zusammensetzungen, die mindestens ein nichtionisches filmbildendes und/oder nichtionisches festigendes Polymer enthalten, umfassend mindestens eine Struktureinheit der Formel (M-I) und mindestens eine Struktureinheit der Formel (M-III) 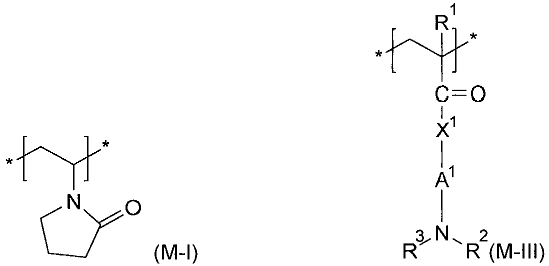 worin
R¹ für ein Wasserstoffatom oder eine Methylgruppe steht,
X¹ für ein Sauerstoffatom oder eine Gruppe NH steht,
A¹ für eine Gruppe Ethan-1,2-diyl, Propan-1,3-diyl oder Butan-1,4-diyl steht
R² und R³ stehen unabhängig voneinander für eine (C₁ bis C₄)-Alkylgruppe.

Dabei ist es insbesondere bevorzugt, wenn das obige nichtionische filmbildende und/oder nichtionische festigende Polymer ausgewählt wird aus mindestens einem Polymer, welches mindestens eines oder mehrere der folgenden Merkmale erfüllt:
- R¹ bedeutet eine Methylgruppe,
- X¹ steht für eine Gruppe NH,
- A¹ steht für Ethan-1,2-diyl oder Propan-1,3-diyl,
- R² und R³, stehen unabhängig voneinander für Methyl oder Ethyl, (besonders bevorzugt für Methyl).

Besonders bevorzugt handelt es sich bei dem nichtionischen filmbildenden und/oder nichtionischen festigenden Polymer dieser Ausführungsform um mindestens ein Polymer, das mindestens eine Struktureinheit der Formel (M-I) und mindestens eine Struktureinheit der Formel (M-III-8) umfasst,

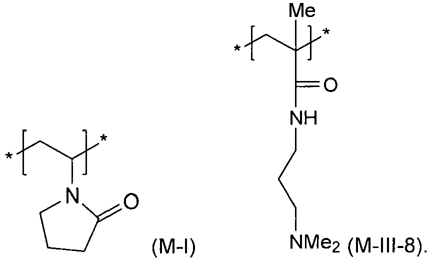

Ein ganz besonders bevorzugtes nichtionisches filmbildendes und/oder nichtionisches festigendes Polymer dieser Ausführungsform ist ein Copolymer aus N-Vinylpyrrolidon und N,N-Dimethylaminiopropylmethacrylamid, welches beispielsweise mit der INCI-Bezeichnung VP/DMAPA Acrylates Copolymer z.B. unter der Handelsbezeichnung Styleze^{®} CC 10 von der Firma ISP verkauft wird.

Bevorzugt sind Mittel zur Behandlung keratinhaltiger Fasern, insbesondere menschlichem Haar, enthaltend in einem kosmetisch akzeptablen Träger
(a) mindestens ein Copolymer umfassend mindestens eine Struktureinheit der Formel (I) und mindestens eine Struktureinheit der Formel (II) 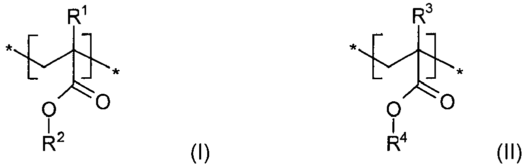 worin
   - R¹ und R³: unabhängig voneinander für ein Wasserstoffatom oder eine Methylgruppe stehen,
   - R²: für eine Dihydroxypropylgruppe (insbesondere für 2,3-Dihydroxypropyl) steht, welche mit einer Glycerylguppe oder einer Oligoglycerylgruppe aus 2 bis 10 Glyceryleinheiten substituiert sein kann
   - R⁴: steht für ein Wasserstoffatom oder eine (C₁ bis C₄)-Alkylgruppe
   und
(b) mindestens ein Copolymer aus Maleinsäureanhydrid und Methylvinylether als filmbildendes und/oder festigendes Polymer und
(c) mindestens ein Esteröl.

Bevorzugt sind Mittel zur Behandlung keratinhaltiger Fasern, insbesondere menschlichem Haar, enthaltend in einem kosmetisch akzeptablen Träger
(a) mindestens ein Copolymer umfassend mindestens eine Struktureinheit der Formel (I) und mindestens eine Struktureinheit der Formel (II) 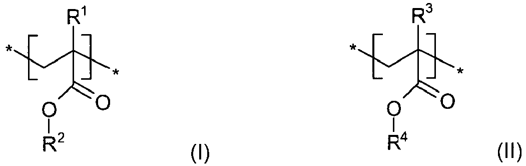 worin
   - R¹ und R³: unabhängig voneinander für ein Wasserstoffatom oder eine Methylgruppe stehen,
   - R²: für eine Dihydroxypropylgruppe (insbesondere für 2,3-Dihydroxypropyl) steht, welche mit einer Glycerylguppe oder einer Oligoglycerylgruppe aus 2 bis 10 Glyceryleinheiten substituiert sein kann
   - R⁴: steht für ein Wasserstoffatom oder eine (C₁ bis C₄)-Alkylgruppe
   und
(b1)mindestens ein Copolymer aus Maleinsäureanhydrid und Methylvinylether
   und
(b2)mindestens ein nichtionisches filmbildendes und/oder nichtionisches festigendes Polymer ausgewählt aus:
   - Polyvinylpyrrolidon,
   - Copolymeren aus N-Vinylpyrrolidon und Vinylestern von Carbonsäuren mit 2 bis 18 Kohlenstoffatomen, insbesondere aus N-Vinylpyrrolidon und Vinylacetat,
   - Copolymere aus N-Vinylpyrrolidon und N-Vinylimidazol und Methacrylamid,
   - Copolymere aus N-Vinylpyrrolidon und N-Vinylimidazol und Acrylamid,
   - Copolymere aus N-Vinylpyrrolidon mit N,N-Di(C₁ bis C₄)-Alkylamino-(C₂ bis C₄)-alkylacrylamid,
   - Copolymere aus N-Vinylpyrrolidon mit N,N-Di(C₁ bis C₄)-Alkylamino-(C₂ bis C₄)-alkylacrylamid,
   oder Gemischen aus diesen Polymeren,
   und
(c) mindestens ein Esteröl.

Bevorzugt sind Mittel zur Behandlung keratinhaltiger Fasern, insbesondere menschlichem Haar, enthaltend in einem kosmetisch akzeptablen Träger
(a) mindestens ein Copolymerisat aus Glycerylacrylat und Acrylsäure,
   und
(b) mindestens ein Copolymer aus Maleinsäureanhydrid und Methylvinylether als filmbildendes und/oder festigendes Polymer
   und
(c) mindestens ein Esteröl.

Bevorzugt sind Mittel zur Behandlung keratinhaltiger Fasern, insbesondere menschlichem Haar, enthaltend in einem kosmetisch akzeptablen Träger
(a) mindestens ein Copolymerisat aus Glycerylacrylat und Acrylsäure,
   und
(b1)mindestens ein Copolymer aus Maleinsäureanhydrid und Methylvinylether
   und
(b2)mindestens ein nichtionisches filmbildendes und/oder nichtionisches festigendes Polymer ausgewählt aus:
   - Polyvinylpyrrolidon,
   - Copolymeren aus N-Vinylpyrrolidon und Vinylestern von Carbonsäuren mit 2 bis 18 Kohlenstoffatomen, insbesondere aus N-Vinylpyrrolidon und Vinylacetat,
   - Copolymere aus N-Vinylpyrrolidon und N-Vinylimidazol und Methacrylamid,
   - Copolymere aus N-Vinylpyrrolidon und N-Vinylimidazol und Acrylamid,
   - Copolymere aus N-Vinylpyrrolidon mit N,N-Di(C₁ bis C₄)-Alkylamino-(C₂ bis C₄)-alkylacrylamid,
   - Copolymere aus N-Vinylpyrrolidon mit N,N-Di(C₁ bis C₄)-Alkylamino-(C₂ bis C₄)-alkylacrylamid,
   oder Gemischen aus diesen Polymeren,
   und
(c) mindestens ein Esteröl.

Im Rahmen einer bevorzugten Ausführungsform enthalten die Zusammensetzungen als filmbildendes und/oder festigendes Polymer mindestens ein filmbildendes anionisches und/oder festigendes anionisches Polymer.

Unter einem anionischen Polymer wird ein Polymer verstanden, das in einem protischen Lösemittel bei Standardbedingungen Struktureinheiten mit anionischen Gruppen, welche durch Gegenionen unter Erhaltung der Elektroneutralität kompensiert werden müssen, trägt und keine Struktureinheiten mit permanent kationischen oder kationisierbaren Gruppen aufweist. Unter anionische Gruppen fallen Carboxyl- und Sulfonsäuregruppen. Diese Polymere sind von den Copolymerisaten der Komponente (a) verschieden.

Die anionischen, filmbildenden und/oder anionischen, festigenden Polymere sind in dem erfindungsgemäßen Mittel bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht der erfindungsgemäßen Zusammensetzung, enthalten.

Es ist bevorzugt, wenn das anionische, filmbildende und/oder anionische, festigende Polymere mindestens eine Struktureinheit der Formel (S1) enthält, die ausgewählt wird aus mindestens einer Struktureinheit der Formeln (S1-1) bis (S1-3)

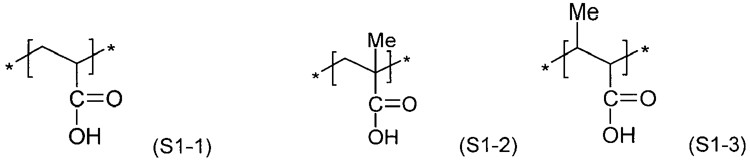

Es ist bevorzugt, wenn das anionische, filmbildende und/oder anionische, festigende Polymere neben mindestens einer Struktureinheit der Formeln (S1-1) bis (S1-3) zusätzlich mindestens eine Struktureinheit der Formel (S2) enthält, die ausgewählt wird aus mindestens einer Struktureinheit der Formeln (S2-1) bis (S2-8) 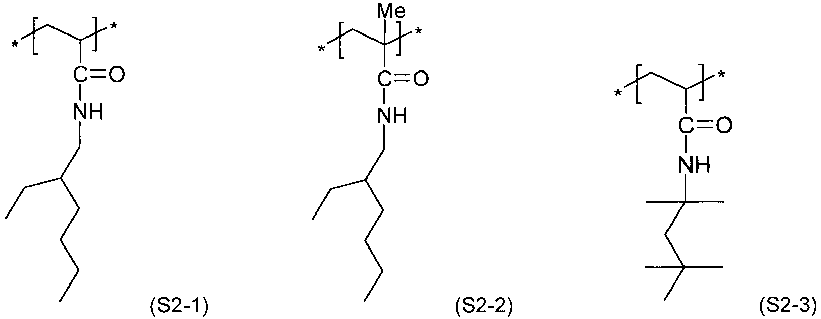 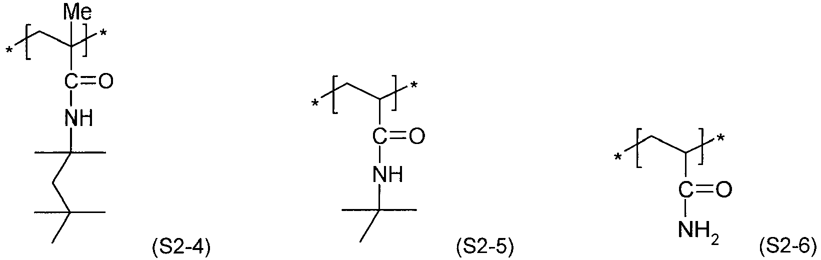 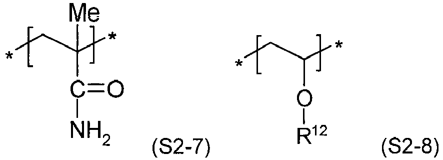 worin
R¹² für eine (C₂ bis C₁₂)-Acylgruppe (insbesondere für Acetyl oder Neodecanoyl) steht.

Im Rahmen einer bevorzugten Ausführungsform gelten solche pulverförmigen Zusammensetzungen als bevorzugt, die als in Form von Partikeln vorliegendes filmbildendes und/oder festigendes Polymer mindestens ein Polymer enthalten, das mindestens eine Struktureinheit der Formel (S1-3) und mindestens eine Struktureinheit der Formel (S2-8) enthalten 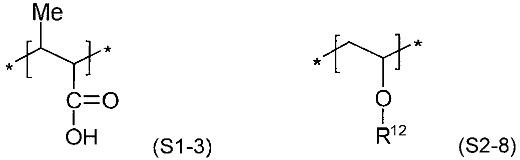 worin R¹² für eine (C₂ bis C₁₂)-Acylgruppe (insbesondere für Acetyl und/oder Neodecanoyl) steht.

Besonders bevorzugte Polymere dieser Art werden ausgewählt aus mindestens einem Polymer der Gruppe, die gebildet wird aus
- Copolymeren aus Vinylacetat und Crotonsäure.
- Copolymeren aus Vinylpropionat und Crotonsäure,
- Copolymeren aus Vinylneodecanoat, Vinylacetat und Crotonsäure.

Solche Copolymere werden beispielsweise von der Firma Clariant unter dem Handelsnamen Aristoflex A 60 (INCI-Bezeichnung: VA/Crotonates Copolymer) in einem Isopropanol-Wasser-Gemisch (60 Gew.-% Aktivsubstanz), von der Firma BASF unter dem Handelsnamen Luviset CA 66 (Vinylacetat/Crotonsäure-Copolymer 90:10, INCI-Bezeichnung VA/Crotonates Copolymer) bereitgestellt, von der Firma National Starch unter dem Handelsnamen Resyn 28-2942 bzw. Resyn 28-2930 (INCI-Bezeichnung: VA/Crotonates/Vinyl Neodecanoate Copolymer) bereitgestellt.

Im Rahmen einer bevorzugten Ausführungsform gelten solche pulverförmigen Zusammensetzungen als erfindungsgemäß bevorzugt, die als anionisches filmbildendes und/oder anionisches festigendes Polymer mindestens ein Polymer enthalten, das mindestens eine Struktureinheit der Formel (S1-1) und mindestes eine Struktureinheit der Formel (S2-5) enthalten

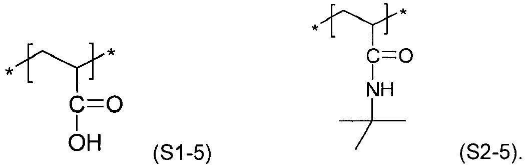

Dabei ist es wiederum besonders bevorzugt, wenn das in Form von Partikeln vorliegende, filmbildende und/oder festigende Polymer neben den obigen Struktureinheiten der Formeln (S1-1) und (S2-5) zusätzlich mindestens eine Struktureinheit der Formel (S3) enthält 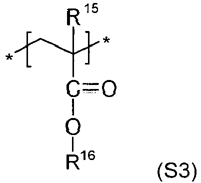 worin
R¹⁵ steht für ein Wasserstoffatom oder eine Methylgruppe
R¹⁶ steht für eine (C₁ bis C₄)-Alkylgruppe (insbesondere eine Methylgruppe oder eine Ethylgruppe).

Besonders bevorzugte Polymere dieser Art werden ausgewählt aus mindestens einem Polymer der Gruppe, die gebildet wird aus Copolymeren aus Acrylsäure und Ethylacrylat und N-tert.-Butylacrylamid. Solche Copolymere werden beispielsweise von der Firma BASF unter dem Handelsnamen Ultrahold^{®} Strong (INCI-Bezeichnung: Acrylates/t-Butylacrylamide Copolymer, weißes, schüttfähiges Granulat) oder Ultrahold^{®} 8 (INCI-Bezeichnung: Acrylates/t-Butylacrylamide Copolymer, weißes, schüttfähiges Granulat) bereitgestellt.

Im Rahmen einer weiteren Ausführungsform enthalten die pulverförmigen Zusammensetzungen als filmbildendes und/oder festigendes Polymer mindestens ein filmbildendes amphoteres und/oder festigendes amphoteres Polymer.

Unter einem amphoteren Polymer wird ein Polymer verstanden, das in einem protischen Lösemittel bei Standardbedingungen Struktureinheiten mit anionischen Gruppen, welche durch Gegenionen unter Erhaltung der Elektroneutralität kompensiert werden müssen, trägt und zusätzlich Struktureinheiten mit durch Protonierung kationisierbaren Gruppen aufweist, jedoch frei von permanent kationisierten Gruppen ist. Unter anionische Gruppen fallen Carboxyl-und Sulfonsäuregruppen. Unter permanent kationisierten Stickstoffatomen sind solche Stickstoffatome zu verstehen, die eine positive Ladung tragen und dadurch eine quartäre Ammoniumverbindung bilden.

Die amphoteren, filmbildenden und/oder amphoteren, festigenden Polymere sind in der erfindungsgemäßen pulverförmigen Zusammensetzung bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht der Zusammensetzung, enthalten.

Es ist geeignet, wenn das amphotere, filmbildende und/oder amphotere, festigende Polymer mindestens eine Struktureinheit der Formel (S1) enthält, die ausgewählt wird aus mindestens einer Struktureinheit der Formeln (S1-1) bis (S1-3)

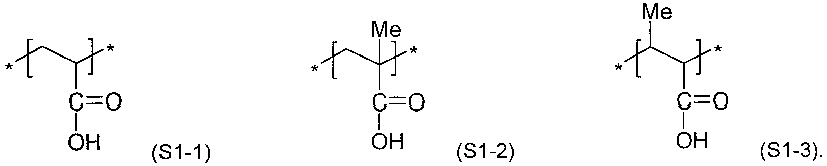

Es ist geeignet, wenn das amphotere, filmbildende und/oder amphotere, festigende Polymere neben mindestens einer Struktureinheit der Formeln (S1-1) bis (S1-3) zusätzlich mindestens eine Struktureinheit der Formel (S2) enthält, die ausgewählt wird aus mindestens einer Struktureinheit der Formel (S2-9) bis (S2-15) 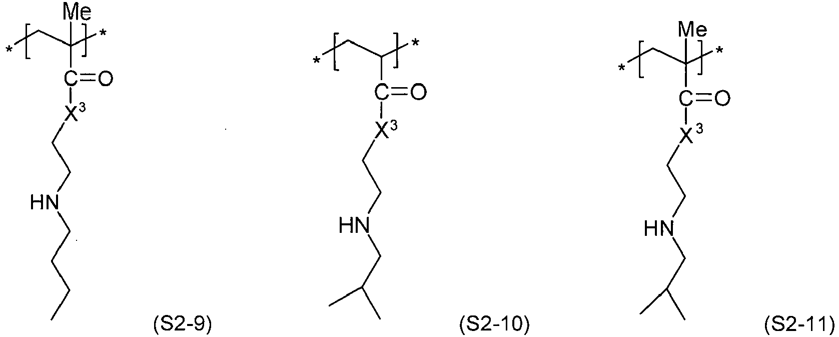 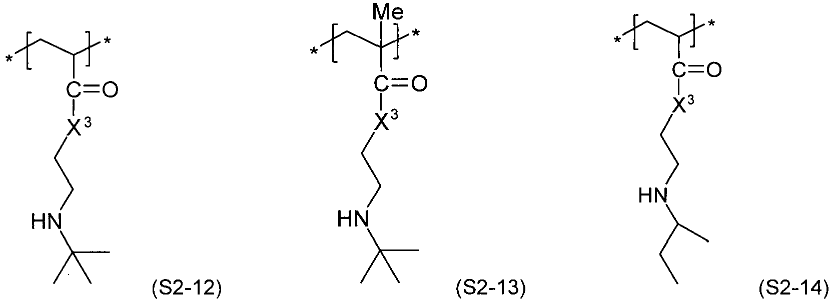 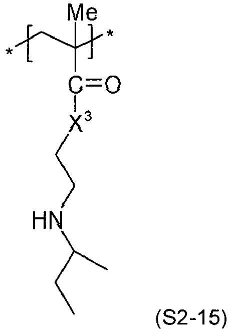 worin
X³ steht für ein Sauerstoffatom oder eine Gruppe NH.

Es ist wiederum geeignet, wenn das amphotere, filmbildende und/oder amphotere, festigende Polymere neben mindestens einer Struktureinheit der Formeln (S1-1) bis (S1-3) und mindestens einer Struktureinheit der Formeln (S2-9) bis (S2-15) zusätzlich mindestens eine Struktureinheit der Formeln (S2-1) bis (S2-8) umfasst 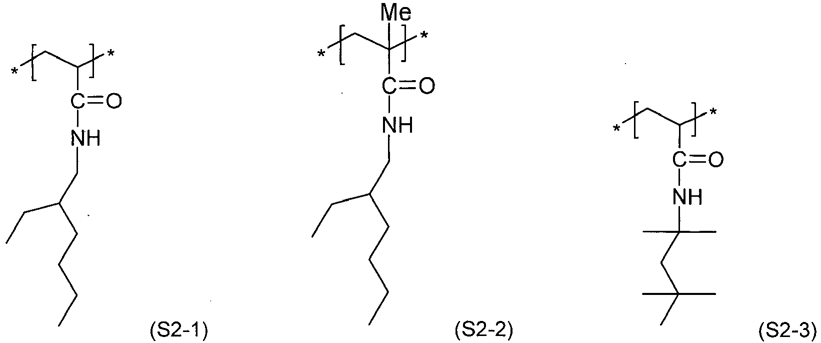 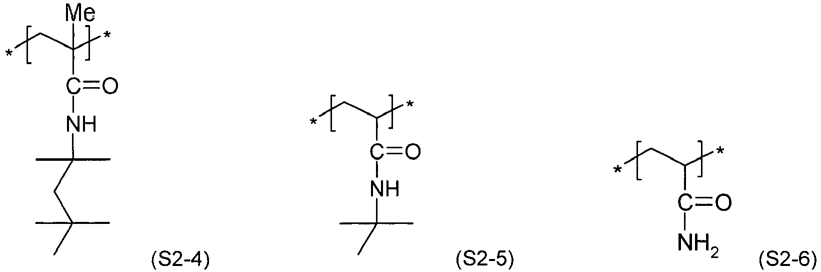 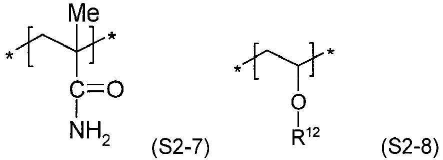 worin
R¹² für eine (C₂ bis C₁₂)-Acylgruppe (insbesondere für Acetyl oder Neodecanoyl) steht.

Im Rahmen einer weiteren Ausführungsform der Erfindung enthält die pulverförmige Zusammensetzung mindestens ein amphoteres, filmbildendes und/oder amphoteres, festigendes Polymer, welches mindestens eine Struktureinheit der Formel (S1-1), mindestens eine Struktureinheit der Formel (S2-3) und mindestens eine Struktureinheit der Formel (S2-16) (insbesondere ausgewählt aus der Gruppe, die gebildet wird aus obigen Formeln (S2-5) bis (S2-12) mit der Maßgabe, dass X³ für ein Sauerstoffatom steht), 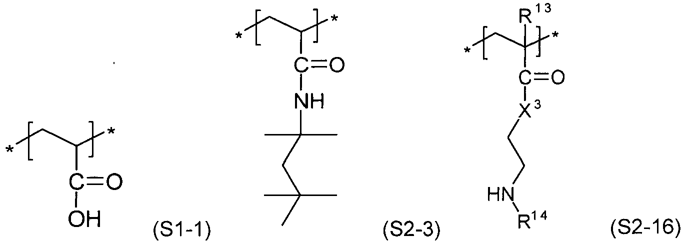
worin X³ steht für ein Sauerstoffatom oder eine Gruppe NH,
R¹³ steht für ein Wasserstoffatom oder eine Methylgruppe und
R¹⁴ steht für eine Alkylgruppe mit 4 Kohlenstoffatomen (insbesondere n-Butyl, sec-Butyl, iso-Butyl oder tert-Butyl).

Dabei ist es wiederum besonders geeignet, wenn das amphotere, filmbildende und/oder anionische, festigende Polymere neben den obigen Struktureinheiten der Formeln (S1-1), (S2-3) und (S2-16) zusätzlich mindestens eine Struktureinheit der Formel (S3) enthält 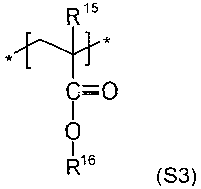 worin
R¹⁵ steht für ein Wasserstoffatom oder eine Methylgruppe
R¹⁶ steht für eine (C₁ bis C₄)-Alkylgruppe (insbesondere eine Methylgruppe oder eine Ethylgruppe).

Bevorzugte Polymere dieser Art werden ausgewählt aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.

Ein Beispiel eines besonders bevorzugt im Rahmen dieser Ausführungsform verwendbaren filmbildenden und/oder festigenden Polymers ist das unter dem Handelsnamen Amphomer^{®} von der Firma National Starch erhältliche Polymer mit der INCI-Bezeichung Octylacrylamide/Acrylates/Butylaminoethylmethacrylate Copolymer.

Gemeinsam mit dem Copolymer der Komponente (a) und dem filmbildenden und/oder festigerndem Polymer der Komponente (b) enthalten die erfindungsgemäßen Mittel mindestens ein Esteröl als Komponente (c).

Esteröle weisen bei einem Druck von 1013 mbar einen Schmelzpunkt kleiner als 30 °C auf und besitzen mindestens eine Alkyloxycarbonyl-Gruppe im Molekül.

Bevorzugte Mittel sind dadurch gekennzeichnet, dass die Esteröle in einer Menge von 0,5 bis 30 Gew.-%, insbeosndere von 1 bis 20 Gew.-%, jeweils bezogen auf das Gewicht des Mittels, enthalten sind.

Bevorzugte Esteröle werden ausgewählt aus Monocarbonsäureestern von (C₃ bis C₂₀)-Alkoholen und/oder Dialkylcarbonaten und/oder Diestern von (C₃ bis C₂₀)-Alkoholen mit alpha, omega-(C₄ bis C₈)-Carbonsäuren

Erfindungsgemäß sind solche Mittel bevorzugt geeignet, in denen dass das Esteröl ausgewählt wird aus mindestens einer Verbindung der Formel (III) und/oder der Formel (IV) 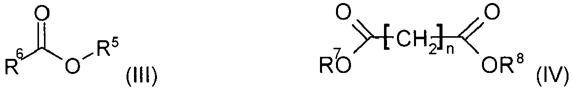 worin
- R⁶: steht für eine lineare (C₃ bis C₁₇)-Alkylgruppe, eine verzweigte (C₃ bis C₁₇)-Alkylgruppe, eine (C₂ bis C₆)-Hydroxyalkylgruppe, eine lineare (C₃ bis C₁₈)-Alkoxygruppe oder eine verzweigte (C₃ bis C₁₈)-Alkoxygruppe,
- R⁵: steht für eine lineare (C₃ bis C₂₀)-Alkylgruppe, verzweigte (C₃ bis C₂₀)-Alkylgruppe
- R⁷ und R⁸: stehen unabhängig voneinander für eine lineare (C₃ bis C₁₀)-Alkylgruppe oder eine verzweigte (C₃ bis C₁₀)-Alkylgruppe,
- n: für eine ganze Zahl von 2 bis 6 steht.

Die erfindungsgemäßen Effekte sind besonders ausgeprägt, wenn das Mittel als Esteröl mindestens eine Verbindung ausgewählt aus Diisopropyladipat, Di-n-butyladipat, Dioctylmaleat, Tridecylneopentanoat (z.B. Ceraphyl^{®} 55 der Firma ISP), Decyloleat (z.B. Ceraphyl^{®} 140 der Firma ISP), Isostearylneopentanoat (z.B. Ceraphyl^{®} 375 der Firma ISP), Isocetylstearat (z.B. Ceraphyl^{®} 494 der Firma ISP), 2-Octyldodecylstearat (z.B. Ceraphyl^{®} ODS der Firma ISP), Isopropylmyristat, Isononansäure-C₁₆-₁₈-alkylester, 2-Ethylhexylpalmitat, Stearinsäure-2-ethylhexylester, Cetyloleat, Kokosfettalkohol-caprinat/-caprylat, n-Butylstearat, Oleylerucat, Isopropylpalmitat, Oleyl Oleate, Laurinsäurehexylester, Cetearyisononanoat, Lauryllactat (z.B. Ceraphyl^{®} 31 der Firma ISP), (C12 bis C15)-Alkyllactat (z.B. Ceraphyl^{®} 41 der Firma ISP), Dihexylcarbonat, Dioctylcarbonat (z.B. Cetiol^{®} CC der Firma Cognis), Didecylcarbonat enthalten ist.

Sehr gute Ergebnisse wurde mit einer Esteröl-Kombination aus
(c1) mindestens einem Esteröl der Formel (III) und/oder der Formel (IV) 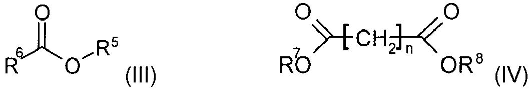 worin
   - R⁶: steht für eine lineare (C₃ bis C₁₇)-Alkylgruppe, eine verzweigte (C₃ bis C₁₇)-Alkylgruppe oder eine (C₂ bis C₆)-Hydroxyalkylgruppe,
   - R⁵: steht für eine lineare (C₃ bis C₂₀)-Alkylgruppe, verzweigte (C₃ bis C₂₀)-Alkylgruppe,
   - R⁷ und R⁸: stehen unabhängig voneinander für eine lineare (C₃ bis C₁₀)-Alkylgruppe oder eine verzweigte (C₃ bis C₁₀)-Alkylgruppe,
   - n: für eine ganze Zahl von 2 bis 6 steht.
   mit
(c2) mindestens einem Esteröl der Formel (V) 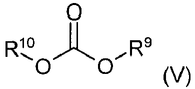 R⁹ und R¹⁰ stehen unabhängig voneinander für eine lineare (C₄ bis C₁₈)-Alkylgruppe oder eine verzweigte (C₄ bis C₁₈)-Alkylgruppe,
erhalten.

Es gelten hierbei jeweils die zuvor genannten bevorzugten Esteröle der Komponente (c) und/oder die bevorzugten Einsatzmengen als bevorzugt.

Als besonders bevorzugtes Esteröl der Komponente (c1) dient mindestens ein Esteröl eines Esters aus linearen oder verzweigten (C₃-C₂₀)-Monocarbonsäuren mit linearen oder verzweigten (C₃ bis C₂₀)-Alkoholen.

Als besonders bevorzugtes Esteröl der Komponente (c2) dient Di(n-octyl)carbonat.

Es ist erfindungsgemäß bevorzugt, wenn das Mittel zusätzlich mindestens ein (C₂ bis C₆)-Polyol enthält. Erfindungsgemäße (C₂ bis C₆)-Polyole enthalten mindestens 2 Hydroxygruppen. Es ist erfindungsgemäß bevorzugt, wenn das (C₂ bis C₆)-Polyol bei 25°C und einem Druck von 1 atm eine Flüssigkeit ist.

Bevorzugt werden die Polyole ausgewählt aus mindestens einem Polyol aus der Gruppe Glycerin, 1,2-Ethylenglykol, 1,2-Propylenglykol, Butan-2,3-diol, 2-Methyl-2,4-butandiol, 2-Methyl-2,4-pentandiol, 1,5-Pentandiol, 2-Ethyl-1,3-hexandiol, 1,2,4-Pentantriol, 1,3,4-Pentandiol, 1,3,5-Pentantriol. Ganz besonders bevorzugt enthalten die erfindungsgemäßen Mittel als Polyol Glycerin. Bevorzugte Mittel enthalten die (C₂ bis C₆)-Polyole (bevorzugt Glycerin) in einer Menge von 0,5 Gew.-% bis 15,0 Gew.-%, besonders bevorzugt von 1,0 Gew.-% bis 10,0 Gew.-%, jeweils bezogen auf das Gewicht des Mittels.

Bevorzugt sind Mittel zur Behandlung keratinhaltiger Fasern, insbesondere menschlichem Haar, enthaltend in einem kosmetisch akzeptablen Träger
(a) mindestens ein Copolymerisat aus Glycerylacrylat und Acrylsäure,
   und
(b) mindestens ein Copolymer aus Maleinsäureanhydrid und Methylvinylether als filmbildendes und/oder festigendes Polymer
   und
(c) mindestens ein Esteröl
   und
(d) Glycerin.

Bevorzugt sind Mittel zur Behandlung keratinhaltiger Fasern, insbesondere menschlichem Haar, enthaltend in einem kosmetisch akzeptablen Träger
(a) mindestens ein Copolymerisat aus Glycerylacrylat und Acrylsäure,
   und
(b1)mindestens ein Copolymer aus Maleinsäureanhydrid und Methylvinylether
   und
(b2)mindestens ein nichtionisches filmbildendes und/oder nichtionisches festigendes Polymer ausgewählt aus:
   - Polyvinylpyrrolidon,
   - Copolymeren aus N-Vinylpyrrolidon und Vinylestern von Carbonsäuren mit 2 bis 18 Kohlenstoffatomen, insbesondere aus N-Vinylpyrrolidon und Vinylacetat,
   - Copolymere aus N-Vinylpyrrolidon und N-Vinylimidazol und Methacrylamid,
   - Copolymere aus N-Vinylpyrrolidon und N-Vinylimidazol und Acrylamid,
   - Copolymere aus N-Vinylpyrrolidon mit N,N-Di(C₁ bis C₄)-Alkylamino-(C₂ bis C₄)-alkylacrylamid,
   - Copolymere aus N-Vinylpyrrolidon mit N,N-Di(C₁ bis C₄)-Alkylamino-(C₂ bis C₄)-alkylacrylamid,
   oder Gemischen aus diesen Polymeren,
   und
(c) mindestens ein Esteröl
   und
(d) Glycerin.

Bevorzugt sind Mittel zur Behandlung keratinhaltiger Fasern, insbesondere menschlichem Haar, enthaltend in einem kosmetisch akzeptablen Träger
(a) mindestens ein Copolymerisat aus Glycerylacrylat und Acrylsäure,
   und
(b) mindestens ein Copolymer aus Maleinsäureanhydrid und Methylvinylether als filmbildendes und/oder festigendes Polymer
   und
(c1) mindestens einem Esteröl der Formel (III) und/oder der Formel (IV) 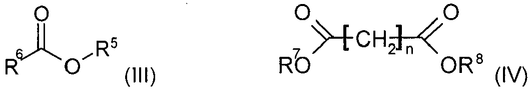 worin
   - R⁶: steht für eine lineare (C₃ bis C₁₇)-Alkylgruppe, eine verzweigte (C₃ bis C₁₇)-Alkylgruppe oder eine (C₂ bis C₆)-Hydroxyalkylgruppe,
   - R⁵: steht für eine lineare (C₃ bis C₂₀)-Alkylgruppe, verzweigte (C₃ bis C₂₀)-Alkylgruppe,
   - R⁷ und R⁸: stehen unabhängig voneinander für eine lineare (C₃ bis C₁₀)-Alkylgruppe oder eine verzweigte (C₃ bis C10)-Alkylgruppe,
   - n: für eine ganze Zahl von 2 bis 6 steht.
   und
(c2) mindestens einem Esteröl der Formel (V) 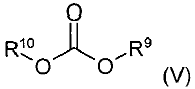
   - R⁹ und R¹⁰: stehen unabhängig voneinander für eine lineare (C₄ bis C₁₈)-Alkylgruppe oder eine verzweigte (C₄ bis C₁₈)-Alkylgruppe.
   und
(d) Glycerin.

Bevorzugt sind Mittel temporären Verformung von Haaren insbesondere menschlichem Haar, enthaltend in einem kosmetisch akzeptablen Träger
(a) mindestens ein Copolymerisat aus Glycerylacrylat und Acrylsäure,
   und
(b1)mindestens ein Copolymer aus Maleinsäureanhydrid und Methylvinylether
   und
(b2)mindestens ein nichtionisches filmbildendes und/oder nichtionisches festigendes Polymer ausgewählt aus:
   - Polyvinylpyrrolidon,
   - Copolymeren aus N-Vinylpyrrolidon und Vinylestern von Carbonsäuren mit 2 bis 18 Kohlenstoffatomen, insbesondere aus N-Vinylpyrrolidon und Vinylacetat,
   - Copolymere aus N-Vinylpyrrolidon und N-Vinylimidazol und Methacrylamid,
   - Copolymere aus N-Vinylpyrrolidon und N-Vinylimidazol und Acrylamid,
   - Copolymere aus N-Vinylpyrrolidon mit N,N-Di(C₁ bis C₄)-Alkylamino-(C₂ bis C₄)-alkylacrylamid,
   - Copolymere aus N-Vinylpyrrolidon mit N,N-Di(C₁ bis C₄)-Alkylamino-(C₂ bis C₄)-alkylacrylamid,
   oder Gemischen aus diesen Polymeren,
   und
(c1) mindestens einem Esteröl der Formel (III) und/oder der Formel (IV) 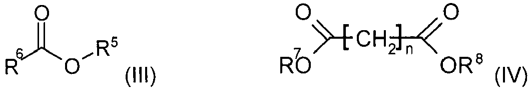 worin
   - R⁶: steht für eine lineare (C₃ bis C₁₇)-Alkylgruppe, eine verzweigte (C₃ bis C₁₇)-Alkylgruppe oder eine (C₂ bis C₆)-Hydroxyalkylgruppe,
   - R⁵: steht für eine lineare (C₃ bis C₂₀)-Alkylgruppe, verzweigte (C₃ bis C₂₀)-Alkylgruppe,
   - R⁷ und R⁸: stehen unabhängig voneinander für eine lineare (C₃ bis C₁₀)-Alkylgruppe oder eine verzweigte (C₃ bis C₁₀)-Alkylgruppe,
   - n: für eine ganze Zahl von 2 bis 6 steht.
   und
(c2) mindestens einem Esteröl der Formel (V) 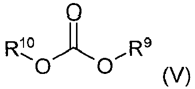
   - R⁹ und R¹⁰: stehen unabhängig voneinander für eine lineare (C₄ bis C₁₈)-Alkylgruppe oder eine verzweigte (C₄ bis C₁₈)-Alkylgruppe
   und
(d) Glycerin.

Im Rahmen einer besonders bevorzugten Ausführungsform liegt das Mittel verdickt vor. Bevorzugte Mittel enthalten als Verdicker zusätzlich mindestens ein Sulfonsäure-Polymer, umfassend mindestens eine Struktureinheit gemäß Formel (P-I) und mindestens eine Struktureinheit gemäß Formel (P-II) 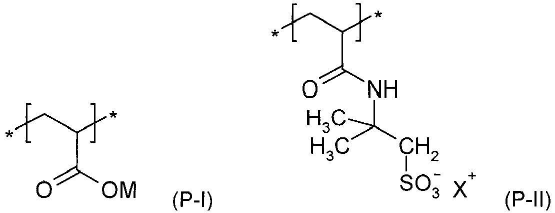 worin M für Wasserstoff oder Natrium und X⁺ für ein physiologisch verträgliches Kation steht.

Das erste Monomer, das im Sulphonsäure-Polymer enthalten ist, ist Acrylsäure oder Natriumacrylat, d.h. das Natriumsalz der Acrylsäure.

Das zweite Monomer, das im Sulphonsäure-Polymer enthalten ist, ist 2-Methyl-2[(1-oxo-2-propenyl)amino]-1-propansulfonsäure (AMPS), die teilweise oder vollständig neutralisiert vorliegen kann. Üblicherweise sind als Kationen Na+ und NH4+ bevorzugt.

Bevorzugte Sulphonsäure-Polymere lassen sich durch die allgemeine Formel 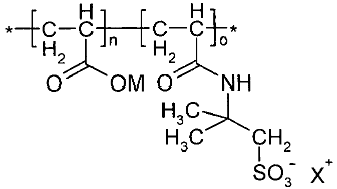 beschreiben, wobei die Indices n und o je nach Molmasse des Polymers variieren und nicht bedeuten sollen, daß es sich zwingend um Blockcopolymere handelt. Vielmehr können Struktureinheiten der Formeln (P-I) und (P-II) im Molekül auch statistisch verteilt vorliegen.

Bevorzugte Sulphonsäure-Polymere dieser Art sind Copolymere aus 2-Methyl-2[(1-oxo-2-propenyl)amino]-1-propansulfonsäure (AMPS) und Natriumacrylat, die beispielsweise als Handelsprodukt Simulgel® EG (INCI-Bezeichnung: Sodium Acrylates/Sodium Acryloyldimethyl Taurate Copolymer, Isohexadecane, Polysorbate 80) der Firma Seppic erhältlich sind.

Es ist bevorzugt, wenn das besagte zusätzliche Sulphonsäure-Polymer zusätzlich zu den Struktureinheiten der Formel (P-I) und (P-II) mindestens eine Struktureinheit der Formel (P-III) umfasst

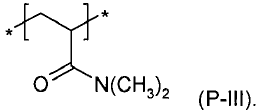

Dieses dritte Monomer ist Dimethylacrylamid.

Diese bevorzugten Sulphonsäure-Polymere lassen sich durch die allgemeine Formel 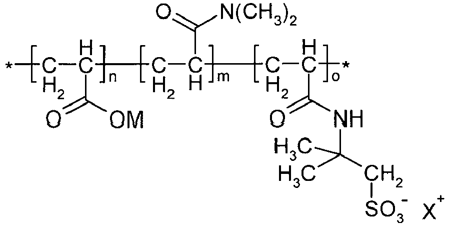 beschreiben, wobei die Indices m und n und o je nach Molmasse des Polymers variieren und nicht bedeuten sollen, daß es sich zwingend um Blockcopolymere handelt. Vielmehr können Struktureinheiten der Formeln (P-I), (P-II) und (P-III) im Molekül auch statistisch verteilt vorliegen.

Die Monomere der Formeln (P-I), (P-II) und (P-III) sind in den Sulphonsäure-Polymer vorzugsweise in solcher Anzahl und Verteilung enthalten, das das Copolymer A Molmassen zwischen 5 und 1000 kDa aufweist. Bevorzugte Mittel sind dadurch gekennzeichnet, daß sie Copolymer(e) A mit Molmassen von 10 bis 750 kDa, vorzugsweise von 25 bis 500 kDa, weiter bevorzugt von 50 bis 400 kDa und insbesondere von 70 bis 250 kDa, enthalten.

Vorzugsweise sind die Monomere der Formeln (P-I), (P-II) und (P-III) innerhalb bestimmter Grenzen im Sulphonsäure-Polymer enthalten. Hier sind erfindungsgemäß bevorzugte Mittel dadurch gekennzeichnet, daß sie Sulphonsäure-Polymere enthalten, die
- 10 bis 90 Mol.-%, vorzugsweise 15 bis 85 Mol.-% und insbesondere 20 bis 80 Mol.-% Monomere der Formel (P-I) und
- 5 bis 85 Mol.-%, vorzugsweise 7,5 bis 80 Mol.-% und insbesondere 10 bis 60 Mol.-% Monomere der Formel (P-II) und
- 5 bis 85 Mol.-%, vorzugsweise 10 bis 80 Mol.-% und insbesondere 15 bis 70 Mol.-% Monomere der Formel (P-III)
enthalten.

Unabhängig davon, ob die Mittel ein oder mehrere Sulphonsäure-Polymere enthalten, ist es bevorzugt, die Sulphonsäure-Polymere innerhalb bestimmter Mengenbereiche einzusetzen. Hier sind erfindungsgemäße Mittel bevorzugt, die die Sulphonsäure-Polymer(e) in einer Menge von 0,1 bis 10 Gew.-%, vorzugsweise 0,5 bis 7,5 Gew.-% und insbesondere 1 bis 5 Gew.-%, jeweils bezogen auf das Gewicht des Mittels, enthalten.

Ganz besonders bevorzugt enthalten die Zusammensetzungen das Sulphonsäure-Polymer in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten (=Sulphonsäure-Polymer), der ein Copolymer darstellt, das mindestens ein Monomer mit einer starken Säurefunktion sowie mindestens ein weiteres Monomer aufweist, das eine schwache Säurefunktion enthält.

Allen vorgenannten, bevorzugt verwendeten inversen Polymerlatices ist gemein, dass sie mindestens ein Öl, bevorzugt ausgewählt aus weißen Mineralölen, Squalan, Isohexadecan und (gegebenenfalls hydriertem) Polyisobuten, sowie mindestens einen Öl-in-Wasser-Emulgator, ausgewählt aus den Ethylenoxid-Addukten von Sorbitanoleat, Ricinusöl, das gewünschtenfalls gehärtet ist, Sorbitanlaurat und Laurylalkohol, sowie weiterhin ausgewählt aus der Polyethylenoxid-freien Öl-in-Wasser-Emulgatorklasse der C6-C22-Fettalkohol-Glucoseether, insbesondere Caprinic Glucoside, Caprylic Glucoside, Capric Glucoside, Lauric Glucoside, Myristic Glucoside, Cetyl Glucoside, Stearyl Glucoside, Arachidyl Glucoside, Behenyl Glucoside, besonders bevorzugt Caprylic Glucoside und Capric Glucoside, enthalten. Demnach sind bevorzugte erfindungsgemäße Zusammensetzungen dadurch gekennzeichnet, dass die Ölphase des inversen Polymerlatex mindestens ein Öl, ausgewählt aus weißen Mineralölen, Squalan, Isohexadecan und (gegebenenfalls hydriertem) Polyisobuten, enthält. Weiter bevorzugte erfindungsgemäße Zusammensetzungen sind dadurch gekennzeichnet, dass der in dem inversen Polymerlatex enthaltene Öl-in-Wasser-Emulgator ausgewählt ist aus den Ethylenoxid-Addukten von Sorbitanoleat, Ricinusöl, das gewünschtenfalls gehärtet ist, Sorbitanlaurat und Laurylalkohol, sowie aus Caprylic Glucoside und Capric Glucoside.

Die bevorzugten einzusetzenden verdickenden Polymerlatices weisen vorzugsweise einen Polymergehalt an Sulphonsäure-Polymer von 30 - 90 Gew.-%, bevorzugt 35 - 75 Gew.-% und besonders bevorzugt 40 - 60 Gew.-%, jeweils bezogen auf den gesamten Latex, auf. Der Polymergehalt des Latex hat dabei jedoch eher nur eine Bedeutung für die Herstellung der erfindungsgemäßen Zusammensetzungen, z. B. in Bezug auf die Mischbarkeit bzw. Dosierbarkeit. Für die Zusammensetzungen selbst ist eher der Polymergehalt an sich, bezogen auf die Zusammensetzung, bedeutsam, siehe oben.

Besonders bevorzugt wird das Sulphonsäure-Polymer in Form eines inversen, auto-inversiblen Polymerlatex eingesetzt, der zusätzlich zum Sulphonsäure-Polymer Isohexadecan als Ölphase und Sorbitanmonostearat als Emulgator enthält.

Bevorzugt sind Mittel zur temporären Verformung von Haaren enthaltend in einem kosmetisch akzeptablen Träger
(a) mindestens ein Copolymerisat aus Glycerylacrylat und Acrylsäure,
   und
(b) mindestens ein Copolymer aus Maleinsäureanhydrid und Methylvinylether als filmbildendes und/oder festigendes Polymer
   und
(c) mindestens ein Esteröl
   und
(d) Glycerin
   und
(e) mindestens ein Sulfonsäure-Polymer, umfassend mindestens eine Struktureinheit gemäß Formel (P-I) und mindestens eine Struktureinheit gemäß Formel (P-II) 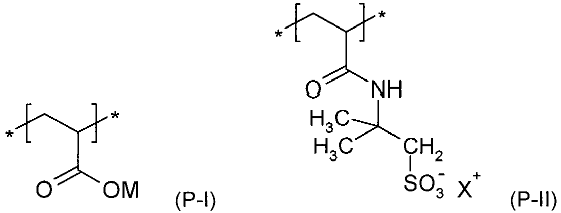 worin M für Wasserstoff oder Natrium und X⁺ für ein physiologisch verträgliches Kation steht.

Bevorzugt sind Mittel zur temporären Verformung von Haaren enthaltend in einem kosmetisch akzeptablen Träger
(a) mindestens ein Copolymerisat aus Glycerylacrylat und Acrylsäure,
   und
(b1)mindestens ein Copolymer aus Maleinsäureanhydrid und Methylvinylether
   und
(b2)mindestens ein nichtionisches filmbildendes und/oder nichtionisches festigendes Polymer ausgewählt aus:
   - Polyvinylpyrrolidon,
   - Copolymeren aus N-Vinylpyrrolidon und Vinylestern von Carbonsäuren mit 2 bis 18 Kohlenstoffatomen, insbesondere aus N-Vinylpyrrolidon und Vinylacetat,
   - Copolymere aus N-Vinylpyrrolidon und N-Vinylimidazol und Methacrylamid,
   - Copolymere aus N-Vinylpyrrolidon und N-Vinylimidazol und Acrylamid,
   - Copolymere aus N-Vinylpyrrolidon mit N,N-Di(C₁ bis C₄)-Alkylamino-(C₂ bis C₄)-alkylacrylamid,
   - Copolymere aus N-Vinylpyrrolidon mit N,N-Di(C₁ bis C₄)-Alkylamino-(C₂ bis C₄)-alkylacrylamid,
   oder Gemischen aus diesen Polymeren,
   und
(c) mindestens ein Esteröl
   und
(d) Glycerin
   und
(e) mindestens ein Sulfonsäure-Polymer, umfassend mindestens eine Struktureinheit gemäß Formel (P-I) und mindestens eine Struktureinheit gemäß Formel (P-II) 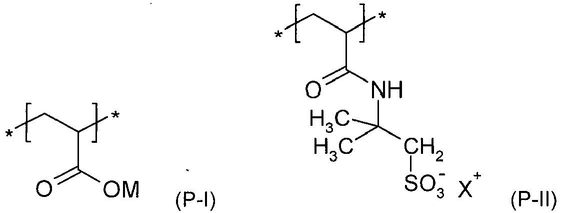 worin M für Wasserstoff oder Natrium und X⁺ für ein physiologisch verträgliches Kation steht.

Bevorzugt sind Mittel zur temporären Verformung von Haaren enthaltend in einem kosmetisch akzeptablen Träger
(a) mindestens ein Copolymerisat aus Glycerylacrylat und Acrylsäure,
   und
(b) mindestens ein Copolymer aus Maleinsäureanhydrid und Methylvinylether als filmbildendes und/oder festigendes Polymer
   und
(c1) mindestens ein Esteröl der Formel (III) und/oder der Formel (IV) 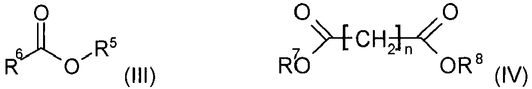 worin
   - R⁶: steht für eine lineare (C₃ bis C₁₇)-Alkylgruppe, eine verzweigte (C₃ bis C₁₇)-Alkylgruppe oder eine (C₂ bis C₆)-Hydroxyalkylgruppe,
   - R⁵: steht für eine lineare (C₃ bis C₂₀)-Alkylgruppe, verzweigte (C₃ bis ₂₀)-Alkylgruppe,
   - R⁷ und R⁸: stehen unabhängig voneinander für eine lineare (C₃ bis C₁₀)-Alkylgruppe oder eine verzweigte (C₃ bis C₁₀)-Alkylgruppe,
   - n: für eine ganze Zahl von 2 bis 6 steht.
   und
(c2) mindestens ein Esteröl der Formel (V) 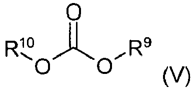
   - R⁹ und R¹⁰: stehen unabhängig voneinander für eine lineare (C₄ bis C₁₈)-Alkylgruppe oder eine verzweigte (C₄ bis C₁₈)-Alkylgruppe.
   und
(d) Glycerin
   und
(e) mindestens ein Sulfonsäure-Polymer, umfassend mindestens eine Struktureinheit gemäß Formel (P-I) und mindestens eine Struktureinheit gemäß Formel (P-II) 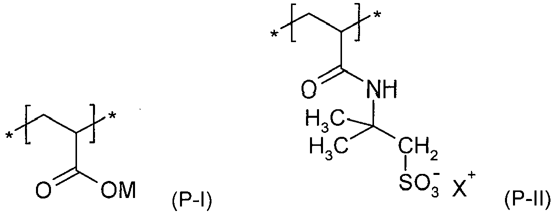 worin M für Wasserstoff oder Natrium und X⁺ für ein physiologisch verträgliches Kation steht.

Bevorzugt sind Mittel zur temporären Verformung von Haaren enthaltend in einem kosmetisch akzeptablen Träger
(a) mindestens ein Copolymerisat aus Glycerylacrylat und Acrylsäure,
   und
(b1)mindestens ein Copolymer aus Maleinsäureanhydrid und Methylvinylether
   und
(b2)mindestens ein nichtionisches filmbildendes und/oder nichtionisches festigendes Polymer ausgewählt aus:
   - Polyvinylpyrrolidon,
   - Copolymeren aus N-Vinylpyrrolidon und Vinylestern von Carbonsäuren mit 2 bis 18 Kohlenstoffatomen, insbesondere aus N-Vinylpyrrolidon und Vinylacetat,
   - Copolymere aus N-Vinylpyrrolidon und N-Vinylimidazol und Methacrylamid,
   - Copolymere aus N-Vinylpyrrolidon und N-Vinylimidazol und Acrylamid,
   - Copolymere aus N-Vinylpyrrolidon mit N,N-Di(C₁ bis C₄)-Alkylamino-(C₂ bis C₄)-alkylacrylamid,
   - Copolymere aus N-Vinylpyrrolidon mit N,N-Di(C₁ bis C₄)-Alkylamino-(C₂ bis C₄)-alkylacrylamid,
   oder Gemischen aus diesen Polymeren,
   und
(c1) mindestens ein Esteröl der Formel (III) und/oder der Formel (IV) 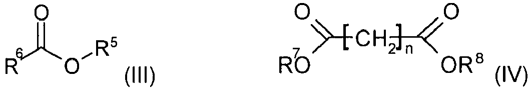 worin
   - R⁶: steht für eine lineare (C₃ bis C₁₇)-Alkylgruppe, eine verzweigte (C₃ bis C₁₇)-Alkylgruppe oder eine (C₂ bis C₆)-Hydroxyalkylgruppe,
   - R⁵: steht für eine lineare (C₃ bis C₂₀)-Alkylgruppe, verzweigte (C₃ bis C₂₀)-Alkylgruppe,
   - R⁷ und R⁸: stehen unabhängig voneinander für eine lineare (C₃ bis C₁₀)-Alkylgruppe oder eine verzweigte (C₃ bis C₁₀)-Alkylgruppe,
   - n: für eine ganze Zahl von 2 bis 6 steht.
   und
(c2) mindestens ein Esteröl der Formel (V) 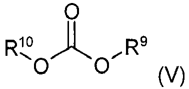
   - R⁹ und R¹⁰: stehen unabhängig voneinander für eine lineare (C₄ bis C₁₈)-Alkylgruppe oder eine verzweigte (C₄ bis C₁₈)-Alkylgruppe
   und
(d) Glycerin
   und
(e) mindestens ein Sulfonsäure-Polymer, umfassend mindestens eine Struktureinheit gemäß Formel (P-I) und mindestens eine Struktureinheit gemäß Formel (P-II) 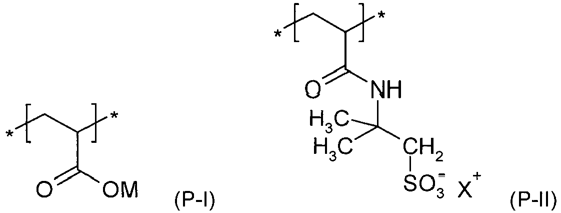 worin M für Wasserstoff oder Natrium und X⁺ für ein physiologisch verträgliches Kation steht.

Dabei sind wiederum die bevorzugten Ausführungsformen der Sulphonsäure-Polymere der Komponente (e) sowie die bevorzugten Einsatzmengen bevorzugt.

Die Mittel können zusätzlich mindestens ein Tensid enthalten, wobei sich prinzipiell nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben.

Die zusätzlichen Tenside sind in dem erfindungsgemäß Mittel bevorzugt in einer Menge von 0,01 Gew.-% bis 5 Gew.-%, besonders bevorzugt von 0,05 Gew.-% bis 0,5 Gew.-%, jeweils bezogen auf das Gewicht des Mittels, enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 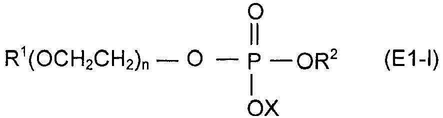 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Die Mittel enthalten die Inhalts- bzw- Wirkstoffe in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind Propylenglykol, Polyethylenglykol, Polypropylenglykol, unverzweigte oder verzweigte Kohlenwasserstoffe wie Pentan, Hexan, Isopentan und cyclische Kohlenwasserstoffe wie Cyclopentan und Cyclohexan.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die Mittel können weiterhin die Hilfs- und Zusatzstoffe enthalten, die üblicherweise herkömmlichen Stylingmitteln zugesetzt werden. Als geeignete Hilfs- und Zusatzstoffe sind insbesondere zusätzliche Pflegestoffe zu nennen.

Als Pflegestoff wird bevorzugt mindestens ein Silikonöl und/oder mindestens ein Silikongum eingesetzt. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Silicium-organischer Verbindungen. Zunächst werden hierunter die Dimethiconole (S1) verstanden. Diese können sowohl linear als auch verzweigt als auch cyclisch oder cyclisch und verzweigt sein.

Besonders geeignete Silikone sind aminofunktionelle Silikone, insbesondere die Silikone, die unter der INCI-Bezeichnung Amodimethicone zusammengefasst sind. Daher ist es erfindungsgemäß bevorzugt, wenn die erfindungsgemäßen Mittel zusätzlich mindestens ein aminofunktionelles Silikon enthalten. Darunter sind Silikone zu verstehen, welche mindestens eine, gegebenenfalls substituierte, Aminogruppe aufweisen.

Die Mittel enthalten die Silikone bzw. Silikongums bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Proteinhydrolysate können sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Proteinhydrolysate pflanzlichen Ursprungs, z. B. Soja-, Mandel-, Erbsen-, Kartoffel- und Weizenproteinhydrolysate, sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das Mittel weiterhin mindestens ein Vitamin, ein Provitamin, eine Vitaminvorstufe und/oder eines derer Derivate enthalten.

Dabei sind solche Vitamine, Provitamine und Vitaminvorstufen bevorzugt, die üblicherweise den Gruppen A, B, C, E, F und H zugeordnet werden.

Bevorzugt enthalten die Mittel Vitamine, Provitamine und Vitaminvorstufen aus den Gruppen A, B, C, E und H.

Panthenol, Pantolacton, Pyridoxin und seine Derivate sowie Nicotinsäureamid und Biotin sind besonders bevorzugt.

Ganz besonders bevorzugt wird als Pflegestoff D-Panthenol, gegebenenfalls in Kombination mit mindestens einem der oben genannten Silikonderivate eingesetzt.

Der Zusatz von Panthenol erhöht die Flexibilität des bei Anwendung des Mittels gebildeten Polymerfilms. Wird also ein besonders flexibler Halt gewünscht, können die Mittel zusätzlich Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die Mittel weiterhin mindestens einen Pflanzenextrakt enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Hinsichtlich der bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind. Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Auch Mono- bzw. Oligosaccharide können als Pflegestoff in den Mitteln eingesetzt werden.

Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten. Ganz besonders bevorzugt eingesetzt wird Glucose, die sowohl in der D-(+)- oder L-(-)- Konfiguration oder als Racemat geeignet ist. Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside. Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten. Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben. Die erfindungsgemäßen Mittel enthalten die Lipide bevorzugt in Mengen von 0,01 - 10 Gew.-%, insbesondere 0,1 - 5 Gew.-%, bezogen auf die gesamte Anwendungszubereitung.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Direktziehende Farbstoffe sind üblicherweise Nitrophenylendiamine, Nitroaminophenole, Azofarbstoffe, Anthrachinone oder Indophenole. Die Mittel gemäß dieser Ausführungsform enthalten die direktziehenden Farbstoffe bevorzugt in einer Menge von 0,001 bis 20 Gew.-%, bezogen auf das gesamte Mittel.

Vorzugsweise liegen im Rahmen einer weiteren Ausführungsform die Mittel in Form einer Creme oder eines Gels, insbesondere als Gel, vor.

Dabei ist es wiederum bevorzugt, wenn in die Creme bzw. das Gel Gasblasen eingearbeitet sind. Diese Gasblasen sind für das menschliche Auge sichtbar. Als Gase eignen sich beispielsweise Luft, Stickstoff, Sauerstoff, Kohlendioxyd, Distickstoffmonoxyd, Argon.

Die creme- bzw. gelförmigen Mittel weisen bevorzugt eine Viskosität von 10000 bis 500000 mPas, besonders bevorzugt von 30000 bis 300000 mPas, (jeweils gemessen mit Brookfield RVDV II+ mit Heilpath, Spindel T-E, 5 rpm, 20°C) auf.

Liegt das Mittel in Form eines Gels vor, so handelt es sich besonders bevorzugt um ein transparentes Gel.

Die Mittel und Produkte, die diese Mittel enthalten, insbesondere Haargele oder Haarcremes, zeichnen sich insbesondere dadurch aus, dass sie dem behandelten Haar einen sehr starken, dauerhaften Frisurenhalt verleihen und das Haar flexibel bleibt. Wird das Mittel als Haargel konfektioniert, resultiert ein Gel mit einer teigigen Konsistenz, das sich dennoch gleichmäßig und ohne zu tropfen auf dem Haar verteilen lässt.

Es ist bevorzugt, das Mittel als leave-on Haarbehandlungsmittel zu verwenden.

Es ist bevorzugt, wenn die keratinhaltigen Fasern vor, während oder nach der Applikation des Mittels in Form gebracht werden.

Ferner gilt es als bevorzugt, das Mittel nicht aus den keratinhaltigen Fasern auszuspülen.

Die nachfolgenden Beispiele sollen den Gegenstand der vorliegenden Erfindung erläutern ohne ihn in irgendeiner Weise zu beschränken.

### Beispiele

Die folgenden Mengenangaben verstehen sich - soweit nichts anderes vermerkt ist - in Gewichtsprozent.

### 1.0 Rezepturen

Es wurden die kosmetischen Mittel A bis I gemäß folgender Tabelle hergestellt:

| | A | B | C | D | E | F | G | H | I |
|---|---|---|---|---|---|---|---|---|---|
| Glycerin | 7,50 | 5,50 | 3,50 | 4,50 | 5,50 | 7,50 | 7,50 | 7,50 | 2,50 |
| Glyceryl Acrylates/Acrylic Acid Copolymer | 0,04 | 0,04 | 0,04 | 0,04 | 0,03 | 0,03 | 0,03 | 0,03 | 0,02 |
| PVM/MA-Copolymer ¹ | 0,03 | 0,03 | 0,03 | 0,03 | 0,02 | 0,02 | 0,02 | 0,02 | 0,01 |
| CeraphylODS ² | 3,00 | 1,50 | 0,50 | 1,50 | - | 1,50 | 1,50 | 1,50 | 1,20 |
| Ceraphyl 55 ³ | 3,00 | 3,00 | - | 3,00 | - | - | - | - | - |
| Ceraphyl 230 ⁴ | - | 1,50 | 1,50 | - | 3,00 | 2,00 | - | 0,50 | - |
| Cetiol CC ⁵ | 4,50 | - | 3,00 | 1,50 | 1,50 | - | 2,00 | 3,50 | 1,00 |
| CETIOL LDO ⁶ | - | - | - | - | 2,00 | - | - | - | - |
| PVP/VA 60/40 W ⁷ | 11,00 | 11,00 | 11,00 | - | 3,00 | 2,00 | 5,00 | - | - |
| Luviskol K 85 ⁸ | - | 5,00 | - | 8,00 | 7,00 | 4,00 | 17,00 | 15,0 | - |
| Luviquat Hold ⁹ | 2,00 | - | - | 3,00 | - | - | - | 5,00 | - |
| Simulgel SMS 88 ¹⁰ | 2,80 | 3,50 | 2,00 | 1,50 | 3,00 | 2,80 | 4,00 | 2,80 | 3,00 |
| Abil Quat 3272 ¹¹ | 1,00 | - | 1,00 | 1,00 | 1,00 | - | 0,10 | 0,10 | 0,50 |
| D-Panthenol | 0,15 | 0,15 | 0,15 | 0,15 | 0,15 | 0,15 | 0,15 | 0,15 | 0,15 |
| PEG-40 Hydr. CastorOil | 0,50 | 0,50 | 0,50 | 0,50 | 0,50 | 0,50 | 0,50 | 0,50 | 0,50 |
| Neolone PE ¹² | 0,60 | 0,60 | 0,60 | 0,40 | 0,60 | 0,60 | 0,60 | 0,60 | 0,60 |
| Phenoxyethanol, rein | - | - | - | 0,30 | - | - | 0,30 | - | - |
| Monomuls 90 0 18 ¹³ | - | - | - | 1,00 | 1,00 | 0,50 | 0,50 | 0,10 | - |
| Cetyltrimethylammoniumchlorid | - | - | - | 0,20 | 0,20 | - | - | - | - |
| Benzophenone-4 | - | - | - | - | - | 0,10 | 0,10 | 0,10 | - |
| Dow Corning 5330 Fluid ¹⁴ | - | 5,00 | - | 2,00 | - | 2,00 | 1,50 | 0,50 | - |
| DOW CORNING 556 ¹⁵ | - | - | - | - | 2,00 | 1,50 | 1,00 | 1,00 | 5,00 |
| Parfum | 0,30 | 0,30 | 0,50 | 0,50 | 0,10 | 0,30 | 0,30 | 0,30 | 0,20 |
| Wasser, vollentsalzt | <---------------------------------------- ad 100 ----------------------------------------> | | | | | | | | |

| | | | | | | | | | |
|---|---|---|---|---|---|---|---|---|---|
| ¹ Copolymer aus Maleinsäureanhydrid und Methylvinylether ² 2-Octyldodecylstearat (INCI-Bezeichnung: Octyldodecyl Stearate) (ISP) ³ Tridecylneopentanoat (INCI-Bezeichnung: Tridecyl Neopentanoate) (ISP) ⁴ Adipinsäuredüsopropylester (INCI-Bezeichnung: Diisopropyladipate) (ISP) ⁵ Dioctylcarbonat (INCI-Bezeichnung: Dicaprylyl Carbonate) (Cognis) ⁶ Gemisch aus Dioctylcarbonat und C₁₂-/C₁₄-Fettalkohol (ca. 40 Gew.-% Fettalkoholanteil) (Cognis) ⁷ Copolymer aus Vinylacetat und N-Vinylpyrrolidon im Verhältnis 40 zu 60 (50 % Aktivsubstanz in Wasser, INCI-Bezeichnung: VP/VA Copolymer) (BASF) ⁸ Polyvinylpyrrolidon (ca. 20% Festkörper in Wasser; INCI-Bezeichnung: PVP) (BASF) ⁹ Copolymer aus N-Vinylpyrrolidon, N-Vinylcaprolactam und quaternisiertem, permanent kationischem N-Vinylimidazol (Methylsulfat) im Gewichtsverhältnis (40/50/10) (20 Gew.-% Polymer gelöst in Wasser, Molekulargewicht ca. 700000 g/mol, Ladungsdichte bei pH 7: 0.8 meq/g; INCI-Bezeichnung: Polyquaternioum-46) (BASF) ¹⁰ Copolymerisat aus Natriumacrylat, Natriumacryloyldimethyltaurat und Dimethylacrylamid (inverse, auto-inversible Latex mit Isohexadecan und Polysorbat-60, ca. 20 Gew.-% Festkörper in Wasser) (Seppic) ¹¹ 3-(3-(3-Cocoamidopropyl)dimethylammonio)-2-hydroxypropyl)propyl-terminiertes Silikon (CAS-Nr.: 134737-05-6; 50 Gew.-% Aktivsubstanz in Wasser; INCI-Bezeichnung: Quaternium-80) (Evonik Goldschmidt) ¹² 2-Methyl-2H-isothiazolin-3-one (ca. 1,55% in 2-Phenoxyethanol; INCI-Bezeichnung: Phenoxyethanol, Methylisothiazolinone) (Rohm & Haas) ¹³ Ölsäuremonoglyzerid (INCI-Bezeichnung: Glyceryl Oleate) (Cognis) ¹⁴ wasserlösliches Polyetherpolysiloxan-Copolymer (INCI-Bezeichnung: PEG/PPG-15/15 Dimethicone) (Dow Corning) ¹⁵ Polyphenylmethylsiloxan (INCI-Bezeichnung: Phenyl Trimethicone) (Dow Corning) | | | | | | | | | |

Die Mittel A bis I ließen sich gut auf dem Haar verteilen, bewirkten eine gute Haarfestigung und zugleich eine hervorragende Haarpflege.

## Patentansprüche

1. Verwendung eines Mittels, enthaltend in einem kosmetisch akzeptablen Träger
(a) mindestens ein Copolymerisat aus Glycerylacrylat und Acrylsäure
(b) mindestens ein filmbildendes und/oder festigendes Polymer und
(c) mindestens ein Esteröl. zur temporären Verformung von Haaren.

2. Verwendung nach Anspruch 1, **dadurch gekennzeichnet, dass** es als filmbildendes und/oder festigendes Polymer
- mindestens ein kationisches filmbildendes und/oder kationisches festigendes Polymer und/oder
- mindestens ein nichtionisches filmbildendes und/oder nichtionisches festigendes Polymer enthält.

3. Verwendung nach einem der Ansprüche 1 oder 2, **dadurch gekennzeichnet, dass** das Esteröl ausgewählt wird aus mindestens einer Verbindung der Formel (III) und/oder der Formel (IV) 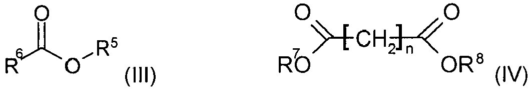 worin
R⁶ steht für eine lineare (C₃ bis C₁₇)-Alkylgruppe, eine verzweigte (C₃ bis C₁₇)-Alkylgruppe, eine (C₂ bis C₆)-Hydroxyalkylgruppe, eine lineare (C₃ bis C₁₈)-Alkoxygruppe oder eine verzweigte (C₃ bis C₁₈)-Alkoxygruppe,
R⁵ steht für eine lineare (C₃ bis C₂₀)-Alkylgruppe, verzweigte (C₃ bis C₂₀)-Alkylgruppe
R⁷ und R⁸ stehen unabhängig voneinander für eine lineare (C₃ bis C₁₀)-Alkylgruppe oder eine verzweigte (C₃ bis C₁₀)-Alkylgruppe,
n für eine ganze Zahl von 2 bis 6 steht.

4. Verwendung nach einem der Ansprüche 1 bis 3, **dadurch gekennzeichnet, dass** es als Esteröl eine Kombination aus
(c1) mindestens einem Esteröl der Formel (III) und/oder der Formel (IV) 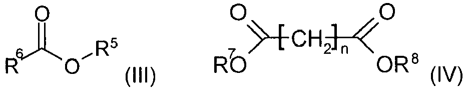 worin
R⁶ steht für eine lineare (C₃ bis C₁₇)-Alkylgruppe, eine verzweigte (C₃ bis C₁₇)-Alkylgruppe oder eine (C₂ bis C₆)-Hydroxyalkylgruppe,
R⁵ steht für eine lineare (C₃ bis C₂₀)-Alkylgruppe, verzweigte (C3 bis C20)-Alkylgruppe,
R⁷ und R⁸ stehen unabhängig voneinander für eine lineare (C₃ bis C₁₀)-Alkylgruppe oder eine verzweigte (C₃ bis C₁₀)-Alkylgruppe,
n für eine ganze Zahl von 2 bis 6 steht.
mit
(c2) mindestens einem Esteröl der Formel (V) 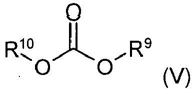
R⁹ und R¹⁰ stehen unabhängig voneinander für eine lineare (C₄ bis C₁₈)-Alkylgruppe oder eine verzweigte (C₄ bis C₁₈)-Alkylgruppe
enthält.

5. Verwendung nach einem der Ansprüche 1 bis 4, **dadurch gekennzeichnet, dass** es die Copolymere (a) in einer Menge von 0,001 Gew.-% bis 1,0 Gew.-%, besonders bevorzugt von 0,005 Gew.-% bis 0,5 Gew.-%, ganz besonders bevorzugt von 0,005 bis 0,1 Gew.%, jeweils bezogen auf das Gewicht des Mittels, enthält.

6. Verwendung nach einem der Ansprüche 1 bis 5, **dadurch gekennzeichnet, dass** die filmbildenden und/oder festigenden Polymere (b) in einer Menge von 0,01 Gew.-% bis 20 Gew.-%, insbesondere von 2,0 Gew.-% bis 10,0 Gew.-%, jeweils bezogen auf das Gewicht des Mittels, enthalten sind.

7. Verwendung nach einem der Ansprüche 1 bis 6, **dadurch gekennzeichnet, dass** die Esteröle in einer Menge von 0,5 bis 30 Gew.-%, insbesondere von 1 bis 20 Gew.-%, jeweils bezogen auf das Gewicht des Mittels, enthalten sind.

8. Verwendung nach einem der Ansprüche 1 bis 7, **dadurch gekennzeichnet, dass** es zusätzlich mindestens ein (C₂ bis C₆)-Polyol (insbesondere Glycerin) enthält.

9. Verwendung nach einem der Ansprüche 1 bis 8, **dadurch gekennzeichnet, dass** es das (C₂ bis C₆)-Polyol in einer Menge von 1,0 bis 10 Gew.-% bezogen auf das Gewicht des Mittels enthält.

10. Verwendung nach einem der Ansprüche 1 bis 9, **dadurch gekennzeichnet, dass** es zusätzlich als Verdicker mindestens ein Sulphonsäure-Polymer enthält, umfassend mindestens eine Struktureinheit gemäß Formel (P-I) und mindestens eine Struktureinheit gemäß Formel (P-II) 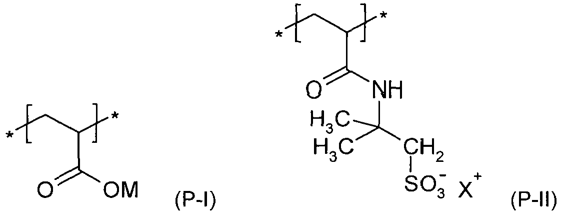 worin M für Wasserstoff oder Natrium und X⁺ für ein physiologisch verträgliches Kation steht.

11. Verwendung nach Anspruch 10, **dadurch gekennzeichnet, dass** es die Sulphonsäure-Polymer(e) in einer Menge von 0,1 bis 10 Gew.-%, vorzugsweise 0,5 bis 7,5 Gew.-% und insbesondere 1 bis 5 Gew.-%, jeweils bezogen auf das Gewicht des Mittels, enthält.

## Claims

1. The use of an agent containing, in a cosmetically acceptable carrier,
(a) at least one copolymer of glyceryl acrylate and acrylic acid,
(b) at least one film-forming and/or setting polymer, and
(c) at least one ester oil
for temporarily reshaping hair.

2. The use according to claim 1, **characterized in that** the agent contains as the film-forming and/or setting polymer
- at least one cationic film-forming and/or cationic setting polymer, and/or
- at least one non-ionic film-forming and/or non-ionic setting polymer.

3. The use according to one of claims 1 or 2, **characterized in that** the ester oil is selected from at least one compound of formula (III) and/or formula (IV), 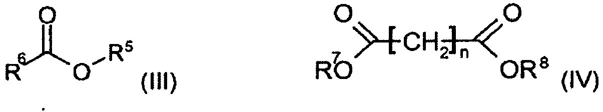 where
R⁶ represents a linear (C₃ to C₁₇) alkyl group, a branched (C₃ to C₁₇) alkyl group, a (C₂ to C₆) hydroxy alkyl group, a linear (C₃ to C₁₈) alkoxy group or a branched (C₃ to C₁₈) alkoxy group,
R⁵ represents a linear (C₃ to C₂₀) alkyl group, branched (C₃ to C₂₀) alkyl group,
R⁷ and R⁸ represent, independently of one another, a linear (C₃ to C₁₀) alkyl group or a branched (C₃ to C₁₀) alkyl group,
n represents an integer from 2 to 6.

4. The use according to one of claims 1 to 3, **characterized in that** the agent contains as the ester oil a combination of
(c1) at least one ester oil of formula (III) and/or formula (IV), 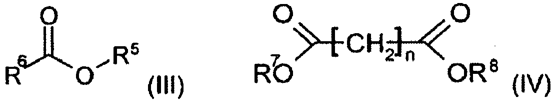 where
R⁶ represents a linear (C₃ to C₁₇) alkyl group, a branched (C₃ to C₁₇) alkyl group or a (C₂ to C₆) hydroxy alkyl group,
R⁵ represents a linear (C₃ to C₂₀) alkyl group, branched (C₃ to C₂₀) alkyl group,
R⁷ and R⁸ represent, independently of one another, a linear (C₃ to C₁₀) alkyl group or a branched (C₃ to C₁₀) alkyl group,
n represents an integer from 2 to 6,
with
(c2) at least one ester oil of formula (V) 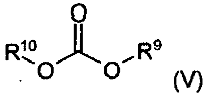
R⁹ and R¹⁰ represent, independently of one another, a linear (C₄ to C₁₈) alkyl group or a branched (C₄ to C₁₈) alkyl group.

5. The use according to one of claims 1 to 4, **characterized in that** the agent contains the copolymers (a) in an amount of 0.001 wt.% to 1.0 wt.%, particularly preferably 0.005 wt.% to 0.5 wt.%, more particularly preferably 0.005 wt.% to 0.1 wt.%, based on the weight of the agent in each case.

6. The use according to one of claims 1 to 5, **characterized in that** the film-forming and/or setting polymers (b) are contained in an amount of 0.01 wt.% to 20 wt.%, in particular 2.0 wt.% to 10.0 wt.%, based on the weight of the agent in each case.

7. The use according to one of claims 1 to 6, **characterized in that** the ester oils are contained in an amount of 0.5 to 30 wt.%, in particular 1 to 20 wt.%, based on the weight of the agent in each case.

8. The use according to one of claims 1 to 7, **characterized in that** the agent additionally contains at least one (C₂ to C₆) polyol (in particular glycerol).

9. The use according to one of claims 1 to 8, **characterized in that** the agent contains the (C₂ to C₆) polyol in an amount of 1.0 to 10 wt.%, based on the weight of the agent.

10. The use according to one of claims 1 to 9, **characterized in that** the agent additionally contains at least one sulfonic acid polymer as a thickener, comprising at least one structural unit according to formula (P-I) and at least one structural unit according to formula (P-II), 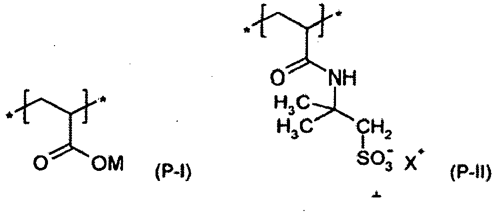 where M represents hydrogen or sodium and X⁺ represents a physiologically acceptable cation.

11. The use according to claim 10, **characterized in that** the agent contains the sulfonic acid polymer(s) in an amount of 0.1 to 10 wt.%, preferably 0.5 to 7.5 wt.% and in particular 1 to 5 wt.%, based on the weight of the agent in each case.

## Revendications

1. Utilisation d'un agent contenant dans un support cosmétiquement acceptable
(a) au moins un copolymère d'acrylate de glycéryle et d'acide acrylique
(b) au moins un polymère fixant et/ou filmogène et
(c) au moins une huile d'ester
pour mettre en forme temporairement des cheveux.

2. Utilisation selon la revendication 1, **caractérisé en ce que** l'agent contient comme polymère fixant et/ou filmogène
- au moins un polymère fixant filmogène et/ou filmogène cationique et/ou
- au moins un polymère fixant non ionique et/ou filmogène non ionique.

3. Utilisation selon l'une des revendications 1 ou 2, **caractérisé en ce que** l'huile d'ester est choisi parmi au moins un composé de la formule (III) et/ou de la formule (IV) 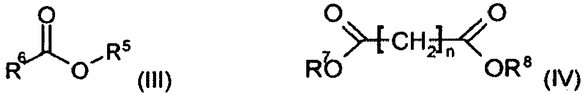 où
R⁶ représente un groupe alkyle en (C₃ à C₁₇) linéaire, un groupe alkyle en (C₃ à C₁₇) ramifié, un groupe hydroxyalkyle en C₂ à C₆, un groupe alcoxy en (C₃ à C₁₈) linéaire ou un groupe alcoxy en (C₃ à C₁₈) ramifié,
R⁵ représente un groupe alkyle en (C₃ à C₂₀) linéaire, un groupe alkyle en (C₃ à C₂₀) ramifié, R⁷ et R⁸ représentent chacun indépendamment un groupe alkyle en (C₃ à C₁₀) linéaire ou un groupe alkyle en (C₃ à C₁₀) ramifié,
n représente un nombre entier de 2 à 6.

4. Utilisation selon l'une des revendications 1 à 3, **caractérisée en ce que** l'agent contient comme huile d'ester une combinaison de
(c1) au moins une huile d'ester de la formule (III) et/ou de la formule (IV) 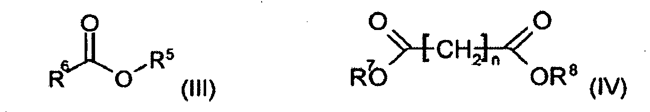 où
R⁶ représente un groupe alkyle en (C₃ à C₁₇) linéaire, un groupe alkyle en (C₃ à C₁₇) ramifié, ou un groupe hydroxyalkyle en C₂ à C₆,
R⁵ représente un groupe alkyle en (C₃ à C₂₀) linéaire, un groupe alkyle en (C₃ à C₂₀) ramifié,
R⁷ et R⁸ représentent chacun indépendamment un groupe alkyle en (C₃ à C₁₀) linéaire ou un groupe alkyle en (C₃ à C₁₀) ramifié,
n représente un nombre entier de 2 à 6,
avec
(c2) au moins une huile d'ester de la formule (V) 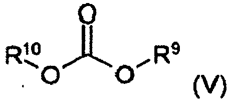 R⁹ et R¹⁰ représentent indépendamment un groupe alkyle en (C₄ à C₁₈) ou un groupe alkyle en (C₄ à C₁₈) ramifié.

5. Utilisation selon l'une des revendications 1 à 4, **caractérisé en ce que** l'agent contient les copolymères (a) dans une quantité allant de 0,001% à 1,0% en poids, plus préférablement de 0,005% à 0,5% en poids, de façon plus particulièrement préférée de 0,005 à 0,1% en poids, à chaque fois sur la base du poids de l'agent.

6. Utilisation selon l'une des revendications 1 à 5, **caractérisé en ce que** les polymères fixants et/ou filmogènes (b) sont contenus dans une quantité allant de 0,01 à 20% en poids, en particulier de 2,0% à 10,0% en poids, à chaque fois sur la base du poids de l'agent.

7. Utilisation selon l'une des revendications 1 à 6, **caractérisée en ce que** les huiles d'ester sont contenues dans une quantité allant de 0,5 à 30% en poids, en particulier de 1 à 20% en poids, à chaque sur la base du poids de l'agent.

8. Utilisation selon l'une des revendications 1 à 7, **caractérisée en ce que** l'agent contient en outre au moins un polyol en C₂ à C₆ (en particulier du glycérol).

9. Utilisation selon l'une des revendications 1 à 8, **caractérisée en ce que** l'agent contient le polyol en C₂ à C₆ dans une quantité de 1,0 à 10% en poids, sur la base du poids de l'agent.

10. Utilisation selon l'une des revendications 1 à 9, **caractérisée en ce que** l'agent contient en outre comme épaississant au moins un polymère d'acide sulfonique comportant au moins un motif structural de la formule (P-I) et au moins un motif structural de la formule (P-II) 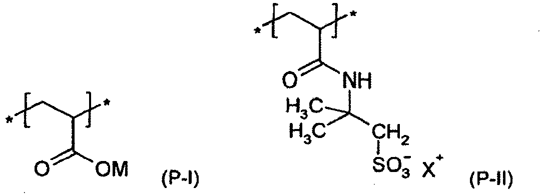 où M représente l'hydrogène ou le sodium et X⁺ représente un cation physiologiquement acceptable.

11. Utilisation selon la revendication 10, **caractérisée en ce que** l'agent contient le ou les polymères d'acide sulfonique dans une quantité allant de 0,1 à 10%, de préférence de 0,5 à 7,5% en poids et en particulier de 1 à 5%, à chaque fois sur la base du poids de l'agent.
